# EUROPÄISCHE PATENTSCHRIFT

(11) **EP 0 696 605 B1**
(45) Veröffentlichungstag und Bekanntmachung des Hinweises auf die Patenterteilung: **20.09.2000**
(21) Anmeldenummer: 95112008.8
(22) Anmeldetag: 31.07.1995
(51) Int. Cl.: C08G 18/42, C08G 18/40, C08G 63/00, C08G 63/06, C08G 64/18, A61L 27/00, A61L 31/00, A61L 17/00

(54) **Biokompatibles Blockcopolymer**
Biocompatible block copolymer
Copolymère séquencé biocompatible

(30) Priorität: 10.08.1994 CH 247894
(43) Veröffentlichungstag der Anmeldung: 14.02.1996
(73) Patentinhaber: Neuenschwander, Peter, 5400 Baden (CH); Uhlschmid, Georg K., 8044 Zürich (CH); Suter, Ulrich W., 8050 Zürich (CH)
(72) Erfinder: Neuenschwander, Peter, CH-5400 Baden (CH); Uhlschmid, Georg K., CH-8044 Zürich (CH); Suter, Ulrich W., CH-8050 Zürich (CH); Ciardelli, Gianluca, CH-8051 Zürich (CH); Hirt, Thomas, CH-8737 Gommiswald (CH); Keiser, Olivier, CH-8004 Zürich (CH); Kojima, Kazushige, 455-Nagoya (JP); Lendlein, Andreas, D-56427 Siershahn (DE); Matter, Sandro, CH-4800 Zofingen (CH)
(74) Vertreter: Patentanwälte Schaad, Balass, Menzl & Partner AG

(56) Entgegenhaltungen:
- EP-A- 0 295 055
- EP-A- 0 552 896
- DE-A- 4 224 401
- GB-A- 2 067 580
- US-A- 4 281 077

## Beschreibung

Die Erfindung betrifft ein biokompatibles Blockcopolymer gemäss dem Oberbegriff von Anspruch 1, ein medizinisches Implantat, das mit dem Blockcopolymer hergestellt worden ist, die Verwendung des Blockcopolymers zur Herstellung eines medizinischen Implantats sowie neue α,ω-Dihydroxypolyester zur Herstellung des Blockcopolymers. Soweit der Ausdruck Medizin verwendet wird, wird darunter sowohl Human- als auch Veterinärmedizin verstanden.

Die Zahl der in der Praxis eingesetzten biokompatiblen Polymere für medizinische Implantate ist überraschend klein. Dies ist, ausser auf das Problem der Verträglichkeit, einerseits auf die hohen technischen Anforderungen bezüglich mechanischer Festigkeit, Sterilisierbarkeit, biologische Abbaubarkeit, etc. andererseits auf die Vielzahl verschiedener administrativer Vorschriften in den einzelnen Ländern zurückzuführen.

Beispielsweise werden für den arteriellen oder venösen Gefässersatz Gefässprothesen, d.h. rohrförmige Implantate mit einem Durchmesser von etwa 1 bis 6 mm, aus nicht-resorbierbaren Materialien wie Dacron, Polytetraflourethylen etc. eingesetzt. Diese Gefässprothesen haben auf ihre Umgebung mehrere negative Wirkungen. Vor allem die negative Veränderung an der Anastomose, d.h. der Verbindungsstelle mit den körpereigenen Gefässen, ist praktisch nicht beeinflussbar. Eine Neubildung einer funktionellen Gefässwand im Bereich der bekannten Implantate ist nicht möglich. Ein Überzug mit funktionellen Gefässzellen zur Vermeidung eines thrombotischen Verschlusses in der Langzeitanwendung ist, wenn überhaupt, nur unter Systemantikoagulation gegeben. Mithin stellen die bekannten Gefässprothesen Fremdkörper dar, deren physikalische und biologische Eigenschaften praktisch nicht beeinflussbar sind.

Durch Beschichtung mit Heparin oder Heparinoiden wurde ein Versuch zur Beeinflussung der biologischen Eigenschaften der Gefässprothesen unternommen. Diese Beschichtungen waren jedoch in qualitativer Hinsicht nur von temporär beschränkter Wirkung.

Aus der GB-A 2 067 580 sind von Caprolacton abgeleitete Polyesterdiole bekannt, die bei der Herstellung von biomedizinischen Trennvorrichtungen eingesetzt werden.

Aus der US-A 4,281,077 sind Blockcopolyester und Blockcourethane bekannt, die als Weichmacher für PVC geeignet sind.

J. Polym. Sci. 13(7) 1353-63 (1975) beschreibt Blockcopolyester mit einem kristallisierenden und einem amorphen Block.

Die US-A 3,663,515 beschreibt die Herstellung von Polyestern und Polyurethanen auf Basis von Polyesterdiolen aus Caprolactonen.

Die DE-A1 42 24 401 beschreibt ein Verfahren, bei dem biologisch abbaubare Polyester mit mehrfunktionellen Verbindungen gespalten werden. Die Spaltprodukte werden durch Polykondensation modifiziert.

Die EP-A1 0 552 896 beschreibt optisch aktive, biologisch abbaubare Blockpolymere, die nur aus einer chemischen Art von Blockbausteinen aufgebaut sind.

Aus der EP-A2 0 295 055 sind biologisch abbaubare Blockcopolymere bekannt, die als Blockbaustein nur L-Milchsäure aufweisen.

Ziel der vorliegenden Erfindung ist die Schaffung eines neuen Polymers mit verbesserten biologischen und verarbeitungstechnischen Eigenschaften.

Dieses Ziel wird durch die kennzeichnenden Merkmale von Anspruch 1 gelöst. Bevorzugte Ausführungsformen der Erfindung bilden den Gegenstand der Ansprüche 2 bis 24.

Das neue biokompatible Multiblockcopolymer ist aus mindestens drei Komponenten aufgebaut und weist mindestens zwei chemisch verschiedene Blockbausteine der im Anspruch 1 definierten Art auf, die mittels Diisocyanat, Disäurehalogenid oder Phosgen miteinander durch lineare Polykondensation verbunden sind. Die Blockbausteine, d.h. die α,ω-Dihydroxy-polyester und α,ω-Dihydroxy-polyether werden nachfolgend auch als Makro-diole und Oligomere bezeichnet. Durch Verknüpfung der Makrodiole mit Diisocyanat werden Polyurethane mit Disäurehalogenid Polyester und mit Phosgen Polycarbonate erhalten.

Eines der Blockbausteine ist ein α,ω-Dihydroxy-polyester, der durch Transesterifikation von Poly-(R)-(3)-hydroxybuttersäure, bzw. deren Copolymeren mit 3-Hydroxyvaleriansäure mit Ethylenglykol erhältlich ist.

Als weitere Makrodiole zur Herstellung der erfindungsgemässen Multiblockcopolymere eignet sich insbesondere eine neue Gruppe von α,ω-Dihydroxy-polyester basierend auf Oligomeren der α-, β-, γ- und ω-Hydroxycarbonsäuren und deren Cooligomeren, die durch ringöffnende Polymerisation von cyclischen Estern oder Lactonen erhalten werden. Bevorzugte cyclische Ester dieser Art sind (L,L)-Dilactid, (D,D)-Dilactid, (D,L)-Dilactid, Diglycolid, oder die bevorzugten Lactame β-(R)-Butyrolacton, β-(S)-Butyrolacton, β-rac-Butyrolacton und ε-Caprolacton oder deren Gemische. Die Ringöffnung erfolgt mit aliphatischen Diolen wie Ethylenglykol oder längerkettigen Diolen. Durch die Menge der Diole wird das Molekulargewicht des erhaltenen Makrodiols bestimmt.

Die ringöffnende Polymerisation der cyclischen Ester oder Lactone erfolgt vorzugsweise in der Masse in Anwesenheit eines Katalysators, beispielsweise SnO(Bu)₂, bei 100°C bis 160°C. Die erhaltenen Makrodiole weisen Molekulargewichte von etwa 300 bis 10'000 Dalton auf. Die aus Gemischen von cyclischen Estern oder Lactonen hergestellten Makrodiole weisen in Abhängigkeit von der Katalysatormenge eine Mikrostruktur auf, in der die Verteilung der monomeren Komponenten zwischen Blockform, statistisch oder alternierend ist. Die Makrodiole und die daraus hergestellten Blockcopolymere weisen in Abhängigkeit von dieser Verteilungsstatistik unterschiedliche physikalische Eigenschaften auf.

Beispiele für Makrodiole, die durch ringöffnende Polymerisation von cyclischen Estern oder Lactonen in Gegenwart eines Katalysators erhalten werden und zur Herstellung der erfindungsgemässen Blockcopolymere verwendet werden können, sind nachfolgend unter a) bis c) aufgeführt.

Die Nomenklatur, wie sie hier verwendet wird, richtet sich nach W.V. Metanomski, Compendium of Macromolecular Nomenklature, Blackwell Scientific Publications (1991). Soweit "ran"-Verteilung aufgeführt ist, werden darunter auch die mit dem Begriff "stat" bezeichneten Verteilungen verstanden.

Die Buchstaben m, n, p, q, r und s in den Formeln a) bis e) stehen für 0 oder eine ganze Zahl von vorzugsweise 1 bis 50.
a) Homo-Makrodiole 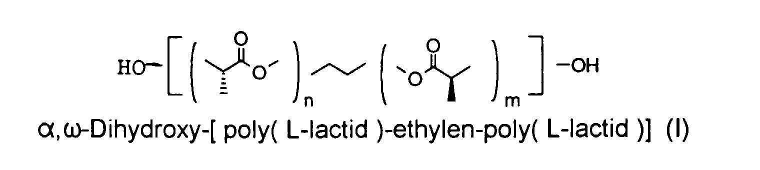
b) Co-Makrodiole aus dem Racemat eines Lactons 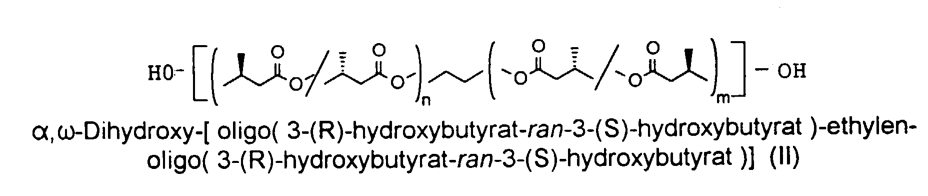
c) Co-Makrodiole, die durch Copolymerisation zweier oder mehrerer verschiedener Lactone und/oder cyclische Diester hergestellt werden.
c₁) Makrodiole, deren Mikrostruktur durch die Menge des Katalysators beeinflusst worden ist: 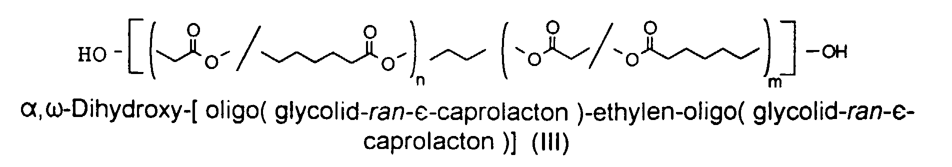 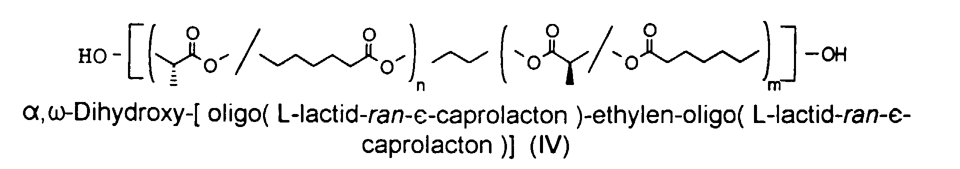 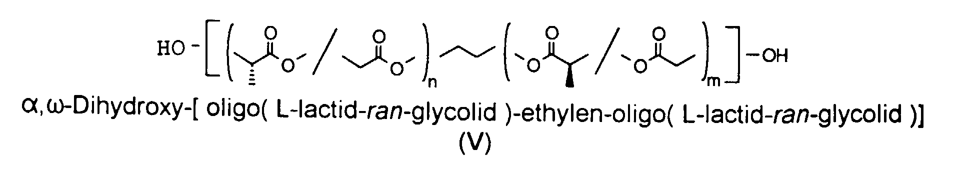
c₂) Makrodiole, deren Mikrostruktur durch die Menge des Katalysators nicht beeinflusst wird: 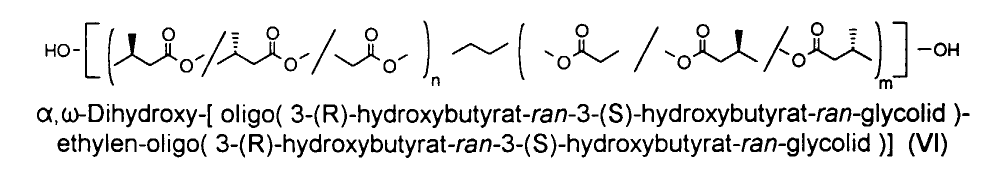 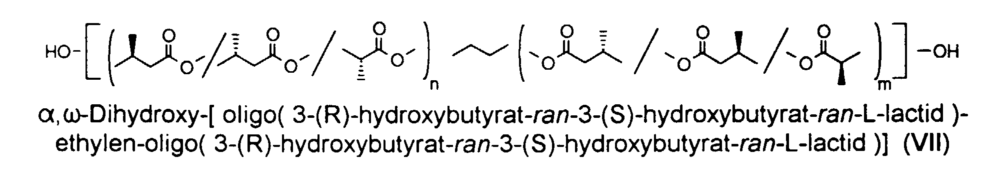 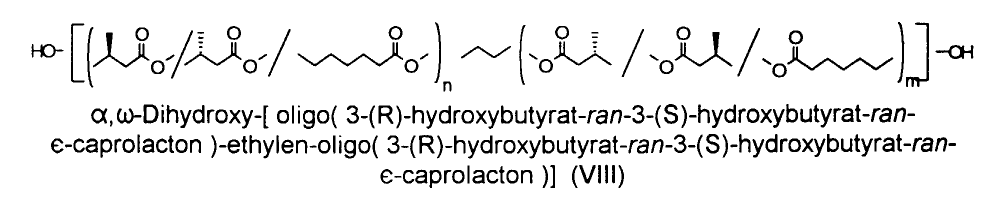
   Die ringöffnende Polymerisation zur Herstellung von Makrodiolen kann auch ohne Katalysator erfolgen. Die nachfolgend dargestellten Makrodiole wurden durch ringöffnende Polymerisation in der Masse bei 100°C bis 160°C ohne Katalysator aus (L,L)-Dilactid, Diglycolid und ε-Caprolacton hergestellt. Sie weisen ein Molekulargewicht in der Grössenordnung von 300 bis 10'000 Dalton auf. Auch hier wird eine Mikrostruktur, d.h. eine charakteristische Verteilung der Monomeren im Polymer beobachtet, wobei die physikalischen Eigenschaften von der Verteilungsstatistik abhängig sind.
d) Homo-Makrodiole 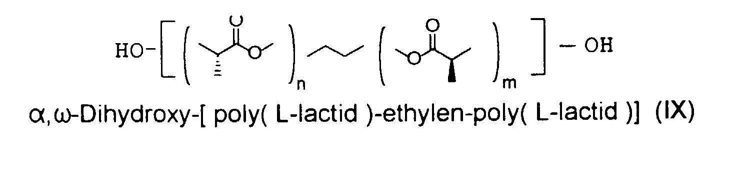
e) Co-Makrodiole mit Blockstruktur 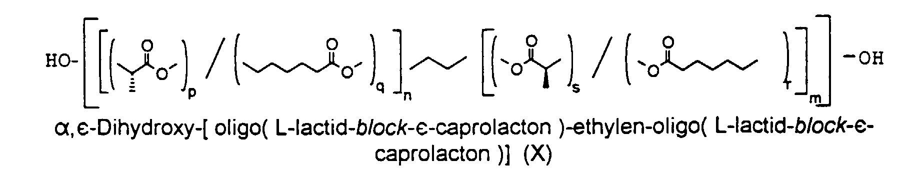 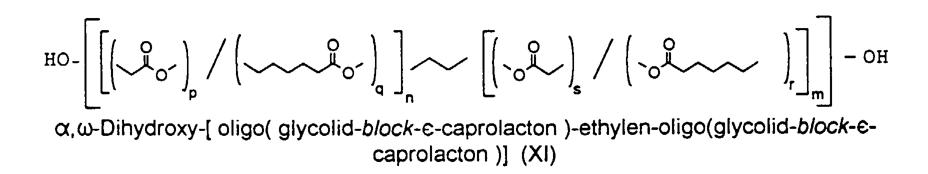 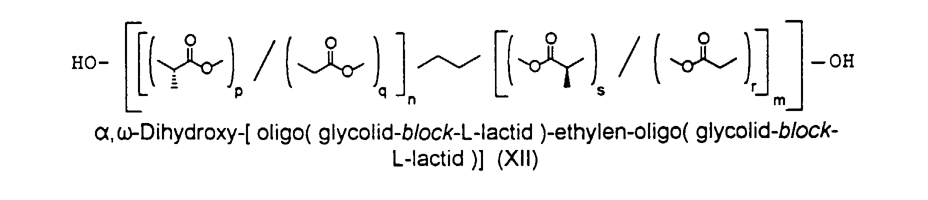
   Das Makrodiol α,ω-Dihydroxy[oligo(3-(R)-hydroxybutyrat)-ethylen-oligo(3-(R)-hydroxybutyrat)] kann alternativ durch Transesterifikation von Poly-((R)-3-hydroxybuttersäure) mit Ethylenglykol vorzugsweise in hoher Konzentration in Diglym bei 135°C in Anwesenheit von Dibutyl-Zinn-Dilaurat als Katalysator hergestellt werden.

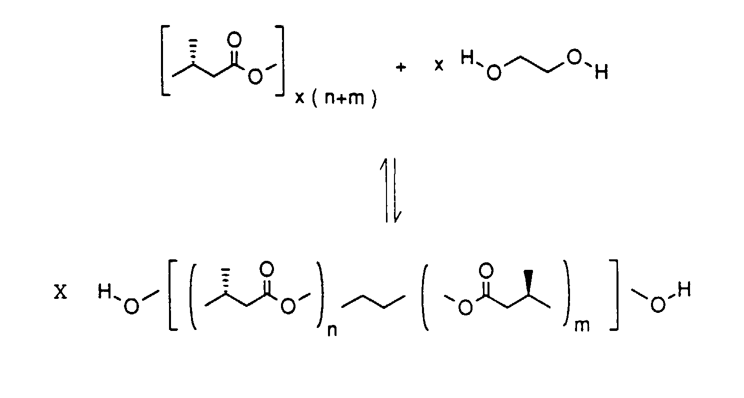

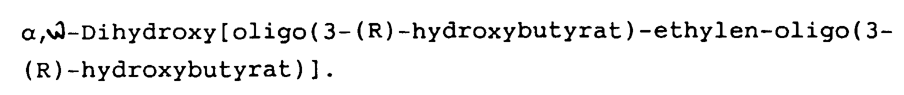

In der gleichen Weise können auch die Copolymeren von 3-(R)-hydroxybuttersäure und 3-Hydroxyvaleriansäure (Biopol mit HV-Gehalt bis ca. 12%) transesterifiziert werden.

Als Diisocyanate für die Herstellung der Polyurethanvariante der erfindungsgemässen Blockcopolymere eignen sich insbesondere Hexamethylendiisocyanat, 2,2,4-Trimethylhexamethylendiisocyanat, Cyclohexyl-1,4-diisocyanat, Cyclohexyl-1,2-diisocyanat, Isophorondiisocyanat, Methylendicyclohexyldiisocyanat und L-Lysindiisocyanatmethylester.

Für die Herstellung der Polyestervariante der erfindungsgemässen Blockcopolymere eignen sich insbesondere die Disäurehalogenide von Oxalsäure, Malonsäure, Bernsteinsäure, Glutarsäure, Adipinsäure, Pimelinsäure, Korksäure, Azelainsäure, Trimethyladipinsäure, Sebazinsäure, Dodecandisäure, Tetradecandisäure und Hexadecandisäure.

Die erfindungsgemässen Blockcopolymere sind aus zwei Arten von Makrodiolen, d.h. Blockbausteinen, aufgebaut, wobei vorzugsweise das eine Makrodiol im Blockcopolymer kristalline Bereiche und das andere Makrodiol amorphe Bereiche im Polymer bildet. Bei den Makrodiolen, die im Blockcopolymer kristalline Bereiche bilden, handelt es sich um solche, die kristallisierbare Verbindungen sind und bei den Makrodiolen, die amorphe Bereiche bilden, kann es sich um kristallisierbare oder nicht-kristallisierbare Verbindungen handeln.

Die erfindungsgemässen Blockcopolymere können weitere cokondensierte niedermolekulare Verbindungen enthalten. Diese weisen zusätzlich zu den für die lineare Cokondensation erforderlichen reaktiven Gruppen, bei denen es sich vorzugsweise um OH- oder NH₂-Gruppen handelt, eine oder mehrere weitere funktionelle Gruppen auf.

Bei diesen funktionellen Gruppen kann es sich um geschützte oder ungeschützte reaktive Gruppen handeln, oder um Gruppen, die den Blockcopolymeren bestimmte Verwendungseigenschaften verleihen. Beispielsweise können diese funktionellen Gruppen die Verwendung der Blockcopolymeren als Röntgenkontrastmittel oder in anderen diagnostischen Verfahren wie CT und MRI,als Mittel zur Kontrasterhöhung ermöglichen.

Wenn es sich bei den funktionellen Gruppen um reaktive Gruppen handelt, ermöglichen sie die Covalentbindung von Wirkstoffen an das erfindungsgemässe Blockcopolymer. Bei solchen Wirkstoffen handelt es sich beispielsweise um Diagnostika, wie Kontrastmittel, pharmazeutische Wirkstoffe, Peptide, Proteine etc..

Besonders geeignete niedermolekulare Comonomere sind:

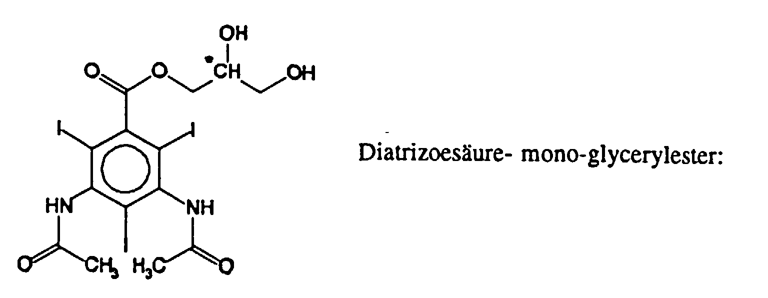

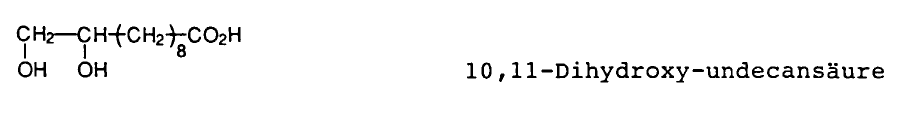

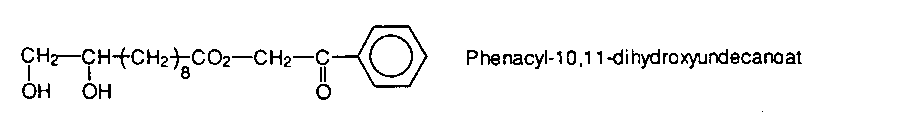

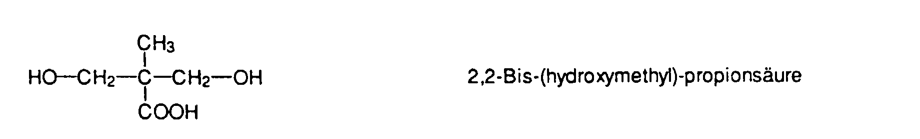

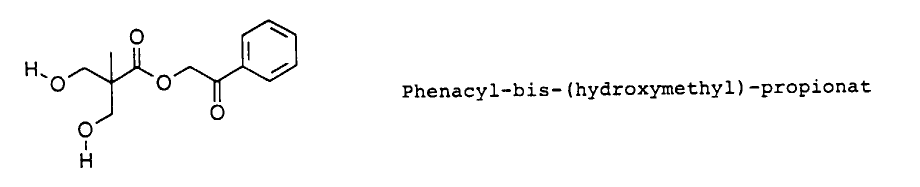

Beispielsweise kann an ein, in den Seitenketten Säuregruppen tragendes Blockcopolymer, das durch Cokondensation von zwei Makrodiolen mit Phenacyl-10,11-dihydroxy-undecanoat und Abspaltung der Phenacylschutzgruppe erhalten wird, das Antibiotikum Doxorubicin gebunden werden. Die Aktivierung der Säuregruppe kann dabei in homogener organischer Lösung durch Steglich-Reagens oder Dicyclohexylcarbodiimid erfolgen.

Zur covalenten Bindung des Peptids RGDS an ein erfindungsgemässes Blockcopolymer kann in einem heterogenen System von Wasser / festes Blockcopolymer ein wasserlösliches Carbodiimid als Aktivator eingesetzt werden.

Die erfindungsgemässen Blockcopolymere sind in organischen Lösungsmitteln löslich und haben den besonderen Vorteil, dass ihre physikalischen, chemischen und biologischen Eigenschaften durch Variieren der Bausteine innerhalb eines breiten Spektrums eingestellt werden können. So können die erfindungsgemässen Blockcopolymere der jeweiligen spezifischen Verwendung angepasst werden. Beispielsweise können die erfindungsgemässen Blockcopolymere, wie es in den Beispielen gezeigt wird, biologisch und sogar im menschlichen oder tierischen Körper abbaubar sein. Die letztgenannte Eigenschaft ist insbesondere für die Verwendung der erfindungsgemässen Blockcopolymere für medizinische Implantate wichtig.

Eine weitere wichtige Eigenschaft der erfindungsgemässen Blockcopolymere ist ihre thermoplastische Verarbeitbarkeit als Folge der linearen Verknüpfung der Grundbausteine. Im allgemeinen sind die Blockcopolymere bei Temperaturen zwischen 80°C bis 200°C, vorzugsweise zwischen 100°C und 140°C, verarbeitbar. Dieser Temperaturbereich bringt für medizinische Implantate den Vorteil der Anpassbarkeit der Form und Grösse des Implantates. Weiterhin kann chirurgisches Nahtmaterial verschweisst werden, was den Verzicht auf das komplizierte Verknüpfen ermöglicht.

Die Implantate können auch in Form eines Rohres vorliegen. Unter einem Rohr werden auch Schläuche verstanden. Die Rohre können runde, eliptische und mehreckige Querschnitte aufweisen, wobei innerhalb eines Rohres auch mehrere Kanäle angeordnet sein können. Bei den erfindungsgemässen Implantaten kann eine Neubildung einer funktionellen Gefässwand oder eines Nervs erfolgen. Durch einen Ueberzug mit funktionellen Gefässzellen kann ein thrombotischer Verschluss in der Langzeitanwendung vermieden werden. Für bestimmte Verwendungen kann das Implantatmaterial, d.h. das Blockcopolymer, eine poröse Struktur aufweisen. Es kann auch Kapselform zur Aufnahme von pharmazeutischen Wirkstoffen oder Diagnostika auch in Form von Partikeln aufweisen.

Nachfolgend werden einige Verwendungen der erfindungsgemässen Blockcopolymere im medizinischen Bereich aufgeführt. Selbstverständlich sind weitere Verwendungen möglich.

Herstellung rohrförmiger Strukturen (Gefässersatz, Luftröhrenersatz, Ersatz anderer biologischer Rohrstrukturen) in fester, spiralförmiger, flexibler, expandierbarer, selbstexpandierender, geflochtener und trikotierter Form, die entsprechend dem biologischen und funktionellen Bedarf an der Innen- resp. Aussenseite, physikalisch und pharmakologisch adequat strukturiert oder beschichtet sein können. Die pharmakologischen Substanzen werden entweder durch Absorption oder covalente chemische Bindung am Blockcopolymer festgehalten. Herstellung von Stents (starr, expandierbar, selbst-expandierend) für Gefässe oder andere biologische Röhrenstrukturen (Oesophagus, Gallenwege, Harnwege).

Herstellung folienförmiger Strukturen (Wundabdeckung, Membranoxygenatoren, Hornhautersatzgrundlage etc.).

Herstellung fadenförmiger Strukturen (chirurgisches Nahtmaterial, Grundlage gewobener, geflochtener oder trikotierter Strukturen).

Herstellung clipförmiger oder klammerförmiger Strukturen für Klammernahtgeräte, Klammern zum Unterbinden kleiner Blutgefässe und Ausnützung der thermoplastischen Eigenschaften zum Verschluss.

Herstellung fester bis gelartiger oder poröser Strukturen als Matrix für die Herstellung von einfachen oder zusammengesetzten biologischen Geweben in vitro (Tissue engeneering) oder in vivo (prekonditionierter Platzhalter für Hautersatz, Fettgewebe, Sehnen, Knorpel und Knochen, Nerven etc.). Anwendung in der topischen Wundbehandlung.

Herstellung polymerer Strukturen, die, auf Grund der physikalischen resp. biologischen Ladungseigenschaften und physikalischen Strukturen (Schäume, Gel, Mikro-Nanosphären) und der Oberflächenstruktur, die Abgabe therapeutisch (Hormone, Medikamente) oder kosmetisch (Liposome, Proteine, Vitamine etc.) über innere anatomische Strukturen oder über die Haut ermöglichen.

Anwendung zur Verödung von Varikocelen, Varicen der Beine, Oeosphagusvarizen) oder von gastro-intestinalen Blutungsquellen (endoskopisch oder transvaskulär).

Herstellung polymerer Strukturen, die in geeigneter Form und Beladung mit bioaktiven Substanzen der reversiblen oder irreversiblen Antikonzeption durch Blockierung (Oviduct, Ductus spermaticus) ermöglichen.

Hertellung künstlicher Gehörknöchelchen (Ossicles).

Verwendung des Blockcopolymers als Grundlage für Züchtung von Hornhautzellen auf Folien zur Transplantion als Hornhautersatz.

Verwendung des Blockcopolymers in entsprechender physikalischer und/oder biologischer Form in der Medizinischen-, Dental-, Mikro- oder Nanotechnologie.

Verwendung in einer Form die in bildgebenden Verfahren (Röntgen, CT, NMR, Chemoembolisation, PET, Mikroskopie) therapeutisch und/oder diagnostisch einsetzbar ist. Die Verarbeitung der erfindungsgemässen Blockcopolymere kann nach bekannten Verfahren durch Extrusion oder Spritzgiessen erfolgen. Folien sind auch durch Verpressen herstellbar. Durch verschiedene bekannte Verfahren, wie Dipcoating oder Phaseninversion bzw. Salzzugabe zur Lösung des Blockcopolymers und Fällung des Polymers können offenporige Strukturen erzeugt werden.

Die mechanischen Eigenschaften der erfindungsgemässen Blockcopolymere sind stark von der Wahl der kristallinen Komponente, deren Molekulargewicht und Massenanteil, sowie der Wahl der nicht-kristallinen Komponente abhängig. Je nach Zusammensetzung können die Blockcopolymere einen E-Modul von 5 MPa bis 2 GPa aufweisen. Es wurden Reissfestigkeiten von 5 bis 20 MPa und Reissdehnungen von 20 bis 900% beobachtet. Die Oberflächenenergien sind ebenfalls von der Zusammensetzung der Copolymeren abhängig. Der Kontaktwinkel mit Wasser, d.h. das Mass für die Oberflächenenergie, liegt zwischen 60° und 85°, d.h. in einem für die Zellhaftung und Proteinbindung günstigen Bereich. Carboxylgruppen tragende Polymeren haben Kontaktwinkel zwischen 40° und 70° gezeigt.

Die Wasseraufnahme der Blockcopolymeren ist auch von deren Zusammensetzung abhängig. Sie liegt im allgemeinen zwischen 0,2 bis 5%, sie kann jedoch in einzelnen Fällen auch höher liegen.

Die erfindungsgemässen Blockcopolymere sind hydrolyseempfindlich. Bei Lagerung in einer Pufferlösung bei 37°C nimmt das Molekulargewicht ab. Die Abbaugeschwindigkeit steigt mit vom pH = 7 abweichendem pH-Wert der Pufferlösung oder auch bei steigender Temperatur.

Die erfindungsgemässen Blockcopolymere erwiesen sich in vitro Zellkulturen mit Makrophagen und Fibroblasten auf Grund der Beobachtung von Zelladhäsion, Zellwachstum, Zellvitalität und Zellaktivierung sowie der Produktion von extrazellulären Proteinen und Zytokinen als biokompatibel. Anhand von in vivo Versuchen bei Ratten in Kurz-, Mittel- und Langzeitversuchen von subkutaner Implantation von Folien und Gelen wurden nur geringfügige Fremdkörperreaktionen bzw. Umkapselung der Implantate beobachtet. Mithin zeigen sich die erfindungsgemässen Blockcopolymere biokompatibel.

In Langzeitversuchen in vivo erwiesen sich die Blockcopolymere als biodegradabel. Die Abbauraten sind stark von der jeweiligen Zusammensetzung und Struktur abhängig.

Nachfolgend wird die Erfindung anhand von Beispielen weiter veranschaulicht.

Die Formeln der Verbindungen der Beispiele sind auf Formelblättern dargestellt. In den Formeln bedeuten die Buchstaben m, m', n, n', p, p', q, q', r, r', s, s', t, u, 0 oder eine ganze Zahl, vorzugsweise von 1 bis 50. x steht für eine ganze Zahl, vorzugsweise von 1 bis 100.

### Beispiel 1

### Herstellung von α,ω-Dihydroxy [oligo(3-(R)-hydroxybutyrat)-ethylen-oligo(3-(R)-hydroxybutyrat)] durch Transesterifizierung von Poly[(R)-3-hydroxybutyrat] mit Ethylenglykol.

1055g Poly[(R)-3-hydroxybutyrat] / Biopol (ICI) werden unter N₂ in 3 l Diglyme bei 140°C gelöst. Dann werden 246 g Ethylenglykol und 5.21 g Dibutylzinn-dilaurat (Kat.) zugegeben. Nach einer Stunde wird 1.5 g (125°C) und nach weiteren 2.5 Stunden nochmals 1.2 g Katalysator zugesetzt. Der Abbau wird durch GPC Messungen ständig verfolgt und in Intervallen von 1 h werden zusätzliche 0,6 g Katalysator zugesetzt bis das angestrebte Molekulargewicht des Abbauprodukts erreicht ist. Kontrolle des Molekulargewichtes durch GPC. Der Abbruch des Abbaus erfolgt durch Ausfällen des Polymers in 10 l Wasser.

Das abgebaute Oligomer wird abfiltriert und insgesamt 5 mal in ca. 6 bis 7 l dest. Wasser aufgeschlämmt und nach 20 h wieder abfiltriert. Nach dem letzten Waschgang wird das körnige Oligomer während einer Stunde trocken gesaugt und danach in 2 grossen Kristallisierschalen zuerst im Trockenschrank bei 50°C im Vakuum getrocknet. Danach im Hochvakuum (10⁻² bar) für 30 Stunden am Trockenschrank bei 60 °C weitergetrocknet.

Das trockene Oligomer wird anschliessend in Methylenchlorid gelöst, so dass eine 30-35% Lösung resultiert. Die leicht erwärmte Lösung wird dann über ein Quarzsandbett auf einer Glasfilternutsche filtriert. Das Filtrat wird chromatographisch über einer Kieselgel 60 - Säule gereinigt.

Säulenhöhe ca. 15 cm, Durchmesser 3 cm. Das Filtrat wird aufkonzentriert, bis Oligomere bei 35°C auszufallen beginnen. Dann wird die Lösung (4,5 l) wurde in 10 l Petrolether 30/50 gegossen, so dass das Oligomer ausfällt.

Der Niederschlag wird abfiltriert und getrocknet.
Ausbeute = 86 % Oligomer (Mₙ = 2450)

### Herstellung von Makrodiolen aus Lactonen unter Verwendung eines Katalysators (Beispiele 2 -4)

### Beispiel 2

### Homooligomere:

### Herstellung von α,ω-Dihydroxy-[poly(L-lactid)ethylen poly(L-lactid)] aus L,L-Dilactid (Halbmikroansatz)

14,4 g (0,1 mol) L,L-Dilactid, 186 mg (3 mmol) Ethylenglycol und 25 mg (0,1 mmol) Dibutylzinnoxid werden in einem 100 ml Zweihalskolben unter N₂ (Qualität 5.0) für 60 Minuten auf 120°C erhitzt.

### Herstellung von α,ω-Dihydroxy-[poly(L-lactid) ethylen poly(L-lactid )] aus L,L-Dilactid (Makroansatz) 100,8 g(0,7 mol)L,L-Dilactid, 1.30 g (21 mmol)

Ethylenglycol und 0.19 g (0,8 mmol) Dibutylzinnoxid werden in einem 500 ml Zweihalskolben unter N₂ (Qualität 5.0) für 60 Minuten auf 120°C erhitzt.

### Aufarbeitung

Das Reaktionsgemisch wird in 20 ml (Makroansatz: 200 ml) 1,2-Dichlorethan gelöst und durch Chromatographie über eine 20-25 cm lange Silicagelsäule gereinigt. Dabei wird die Säule mit Petrolether angesetzt. Als Fließmittel wird 1,2-Dichlorethan verwendet. Nach Einengen des Lösungsmittels wird in fünffachem Volumenüberschuß an Petrolether ausgefällt und anschließend zweimal mit Petrolether gewaschen. Das Produkt wird 3 Tage unter Hausvakuum bei 80°C, dann für einen Tag unter Hochvakuum getrocknet.

### Beispiel 3

### Makrodiole, hergestellt aus dem Racemat eines Lactons Herstellung von α,ω-Dihydroxy-[oligo(3-(R)-hydroxybutyratran-3-(S)-hydroxybutyrat) ethylen oligo(3-(R)-hydroxybutyrat-ran-3-(S)-hydroxybutyrat)] aus rac-β-Butyrolacton (Halbmikroansatz)

8,609 g (0,1 mol) rac-β-Butyrolacton, 182 mg (2,9 mmol) Ethylenglycol und 500 mg (2,0 mmol) Dibutylzinnoxid werden in einem 100 ml Zweihalskolben unter N₂ (Qualität 5.0) für 120 Minuten auf 135°C erhitzt.

### Herstellung von α,ω-Dihydroxy-[oligo(3-(R)-hydroxybutyratran-3-(S)-hydroxybutyrat) ethylen oligo(3-(R)-hydroxybutyrat-ran-3-(S)-hydroxybutyrat)] aus rac-β-Butyrolacton (Makroansatz)

60,3 g (0,7 mol) rac-β-Butyrolacton, 1.30 g (21 mmol) Ethylenglycol und 0,20 g (0,8 mmol) Dibutylzinnoxid werden in einem 500 ml Zweihalskolben unter N₂ (Qualität 5.0) für 120 Minuten auf 135°C erhitzt.

### Aufarbeitung

Das Reaktionsgemisch wird in 20 ml (Makroansatz: 200 ml) 1,2-Dichlorethan gelöst und durch Chromatographie über eine 20-25 cm lange Silicagelsäule gereinigt. Dabei wird die Säule mit Petrolether angesetzt. Als Fließmittel wird 1,2-Dichlorethan verwendet. Nach Abdestillieren des Lösungsmittels wird in fünffachem Volumenüberschuß an Petrolether ausgefällt und anschließend zweimal mit Petrolether gewaschen. Das Produkt wird 3 Tage unter Hausvakuum bei 80°C, dann für drei Tage unter Hochvakuum getrocknet.

### Beispiel 4

### Makrodiole, hergestellt aus zwei oder mehreren Lactonen

### Herstellung von α,ω-Dihydroxy-[oligo(glycolid -ran-ε-caprolacton) ethylen oligo (glycolid-ran-ε-caprolacton)] aus Diglycolid und ε-Caprolacton (Halbmikroansatz)

2,9 g (0,025 mol) Diglycolid, 5,7 g (0,05 mol) ε-Caprolacton, 186 mg (3 mmol) Ethylenglycol und 25 mg (0,1 mmol) Dibutylzinnoxid werden in einem 100 ml Zweihalskolben unter N₂ (Qualität 5.0) für 30 Minuten auf 140°C erhitzt.

### Herstellung von α,ω-Dihydroxy-[oligo(glycolid -ran-ε-caprolacton) ethylen oligo (glycolid-ran-ε-caprolacton )] aus Diglycolid und ε-Caprolacton (Makroansatz)

20,3 g (0,175 mol) Diglycolid, 40,0 g (0,35 mol) ε-Caprolacton, 1.30 g (21 mmol) Ethylenglycol und 174 mg (0,7 mmol) Dibutylzinnoxid werden in einem 500 ml Zweihalskolben unter N₂ (Qualität 5.0) für 30 Minuten auf 140°C erhitzt.

### Aufarbeitung

Das Reaktionsgemisch wird in 20 ml (Makroansatz: 200 ml) 1,2-Dichlorethan gelöst und durch Chromatographie über eine 20-25 cm lange Silicagelsäule gereinigt. Dabei wird die Säule mit Petrolether angesetzt. Als Fließmittel wird 1,2-Dichlorethan verwendet. Nach Abdestillieren des Lösungsmittels wird in fünffachem Volumenüberschuß an Petrolether ausgefällt und anschließend zweimal mit Petrolether gewaschen. Das Produkt wird 3 Tage unter Hausvakuum bei 80°C, dann für 3 Tage unter Hochvakuum getrocknet.

### Herstellung von Makrodiolen ohne Katalysator

### (Beipiele 5 - 6)

### Beispiel 5

### Homooligomere

### Herstellung von α,ω-Dihydroxy-[poly(L -lactid) ethylen poly(L-lactid)] aus L,L-Dilactid (Halbmikroansatz)

14,4 g (0,1 mol) L,L-Dilactid und 186 mg (3 mmol) Ethylenglycol werden in einem 100 ml Zweihalskolben unter N₂ (Qualität 5.0) für 3 Tage auf 120°C erhitzt.

### Herstellung von α,ω-Dihydroxy-[poly(L-lactid) ethylen poly(L-lactid)] aus L,L-Dilactid (Makroansatz)

70,6 g (0,7 mol) L,L-Dilactid und 1.30 g (21 mmol) Ethylenglycol werden in einem 500 ml Zweihalskolben unter N₂ (Qualität 5.0) für 3 Tage auf 120°C erhitzt.

### Aufarbeitung

Das Reaktionsgemisch wird in 20 ml (Makroansatz: 200 ml) 1,2-Dichlorethan gelöst. Anschließend wird in fünffachem Volumenüberschuß an Petrolether bei 0°C ausgefällt und zweimal mit Petrolether gewaschen. Das Produkt wird 3 Tage unter Hausvakuum bei 80°C, dann für einen Tag unter Hochvakuum getrocknet.

### Beispiel 6

### Makrodiole mit Blockstruktur hergestellt aus zwei oder mehreren verschiedenen Lactonen

### Herstellung von α,ω-Dihydroxy-[oligo(glycolid-block-ε-caprolacton ) ethylen oligo(glycolid-block-ε-caprolacton)] aus Diglycolid und ε-Caprolacton (Halbmikroansatz)

2,9 g (0,025 mol) Diglycolid, 5,7 g (0,05 mol) ε-Caprolacton und 186 mg (3 mmol) Ethylenglycol werden in einem 100 ml Zweihalskolben unter N₂ (Qualität 5.0) für 3 Tage auf 140°C erhitzt.

### Herstellung von α,ω-Dihydroxy-[oligo(glycolid -block-ε-caprolacton) ethylen oligo (glycolid -block-ε-caprolacton)] aus Diglycolid und ε-Caprolacton (Makroansatz)

20,3 g (0,0175 mol) Diglycolid, 40,0 g (0,035 mol) ε-Caprolacton und 1.3 mg (21 mmol) Ethylenglycol werden in einem 500 ml Zweihalskolben unter N₂ (Qualität 5.0) für 3 Tage auf 140°C erhitzt.

### Aufarbeitung

Das Reaktionsgemisch wird in 20 ml (Makroansatz: 200 ml) Diglym bei 140°C gelöst. Anschließend wird in fünffachem Volumenüberschuß an Petrolether bei 0°C ausgefällt und zweimal mit Petrolether gewaschen. Das Produkt wird 3 Tage unter Hausvakuum bei 80°C, dann für einen Tag unter Hochvakuum getrocknet.

### Beispiel 7

### Makrodiole aus Polyetherdiol und Diglycolid (ohne Katalysator)

### Herstellung von α,ω-Dihydroxy-[oligo(glycolid )poly (tetrahydrofuran)oligo (glycolid)] aus Poly(tetrahydrofuran) und Diglycolid

### Trocknung von Poly(tetrahydrofuran)

50 g (0,077 mol) Poly(tetrahydrofuran) (Mn=650) werden in 100 ml 1,2-Dichlorethan gelöst und in einem Soxhlet-Extraktor, der mit Molsieb A4 beschickt wird, getrocknet. Das Molsieb wird nach 2 bis 3 Stunden Trocknung jeweils ausgetauscht. Es wird dabei unter Stickstoff gearbeitet. Nach der Trocknung wird das Lösungsmittel abdestilliert.

### Polymerisation mit Diglycolid

50 g (0,077 mol) Poly(tetrahydrofuran) (Mn=650) und 4,47 g (0,077 mol) Diglycolid werden in einem 250 ml Zweihalskolben unter Stickstoff für 3 Tage auf 135°C erhitzt.

### Aufarbeitung

Das Reaktionsgemisch wird aus einer Lösung mit vorgetrocknetem 1,2-Dichlorethan in Hexan umgefällt.

### Beispiel 8

### Makrodiole aus Polyetherdiol und Diglycolid (mit Katalysator)

### Herstellung von α,ω-Dihydroxy-[oligo(glycolid)poly (tetrahydrofuran)oligo(glycolid)] aus Poly(tetrahydrofuran) und Diglycolid mit Katalysator

=

### Trocknung von Poly(tetrahydrofuran)

50 g (0,077 mol) Poly(tetrahydrofuran) (Mn=650) werden in 100 ml 1,2-Dichlorethan gelöst und in einem Soxhlet-Extraktor, der mit Molsieb A4 beschickt wird, getrocknet. Das Molsieb wird nach 2 bis 3 Stunden Trocknung jeweils ausgetauscht. Es wird dabei unter Stickstoff gearbeitet. Nach der Trocknung wird das Lösungsmittel abdestilliert.

### Polymerisation mit Diglycolid

50 g (0,077 mol) Poly(tetrahydrofuran) (Mn=650), 4,47 g (0,077 mol) Diglycolid und 75 mg (0,3 mmol) Dibutylzinnoxid werden in einem 250 ml Zweihalskolben unter Stickstoff für 15 Minuten auf 135°C erhitzt.

### Aufarbeitung

Das Reaktionsgemisch wird in 100 ml 1,2-Dichlorethan (vorgetrocknet) gelöst und durch Chromatographie über eine 20 bis 25 cm lange Silicagelsäule gereinigt. Dabei wird die Säule mit Petrolether angesetzt. Als Fliessmittel wird 1,2-Dichlorethan verwendet. Nach Einengen des Lösungsmittels wird im 5-fachen Überschuss an Petrolether ausgefällt. Das Produkt wird 3 Tage unter Hausvakuum bei 40°C, dann für einen Tag unter Hochvakuum getrocknet.

### Definitionen:

HG3000 (kristallisierendes Segment)* α,ω-Dihydroxy-[oligo(glycolid-*block-ε* -caprolacton) ethylen oligo(glycolid-*block*-ε-caprolacton)], vgl. 1-b-2, mit einer Molmasse M=2479gmol-1 (VPO) und einem Molverhältnis n(Glycolid) : n(ε-Caprolacton) von 2:1

SG3000 (nicht kristallisierendes Segment)* α,ω-Dihydroxy-[oligo(glycolid-ran-ε-caprolacton) ethylen oligo(glycolid-*ran*-ε-caprolacton )], vgl. 2-b-1, mit einer Molmasse M=2475gmol-1 (VPO) und einem Molverhältnis n(Glycolid) : n(ε -Caprolacton ) von 1:1

SG500 (nicht kristallisierendes Segment)* α,ω-Dihydroxy-[oligo(glycolid-*block-ε*-caprolacton) ethylen oligo(glycolid-*block-ε*-caprolacton)], vgl. 1-b-2, mit einer Molmasse M=475gmol-1 (VPO) und einem Molverhältnis n(Glycolid) : n(ε-Caprolacton) von 0.85:0.15

PHB-Diol (kristallisierendes Segment)* α,ω-Dihydroxy-[oligo(3-(R)-hydroxybutyrat) ethylen oligo(3-(R)-hydroxybutyrat)] mit einer Molmasse Mₙ=2300gmol-1 (VPO)

PHB/HV-Diol (kristallisierendes Segment)* α,ω-Dihydroxy-[oligo(3-(R)-hydroxybuttersäure-*co*-3-(R)-hydroxyvaleriansäure)-ethylen-oligo(3-(R)-hydroxybuttersäure-co-3-(R)-hydroxyvaleriansäure)], hergestellt aus Biopol mit 4% Hydroxyvaleriansäure. Mₙ=2300gmol-1

PCL-Diol (kristallines Segment, im Blockcopolymer amorph vorliegend)* α,ω-Dihydroxy-poly(ε-Caprolacton) ethylen-oxy-ethylen-poly(ε-Caprolacton)] mit einer Molmasse M=1200gmol-1 (VPO)

Diorez (Herstseller: Macpherson Polymers) (nicht kristallisierendes Segment)* α,ω-Dihydroxy-<oligo(adipinsäure-*alt-*(butandiol; diethylenglykol; ethylenglykol))>

PTHF-Diol (nicht kristallisierendes Segment)* α,ω-Dihydroxy-poly(tetramethylenoxy) ethylen poly(tetramethylenoxy)] mit einer Molmasse M=650gmol-1 (VPO)
* bei Raumtemperatur

Lysinmethylesterdiisocyanat = 2,6-Diisocyanatcapronsäuremethylester= LDI

2,2,4-Trimethylhexamethylen-1,6-diisocyanat = Isomerengemisch 2,2,4/2,4,4-Isomer ~ 1:1 = TMDI

### Beispiel 9

### Herstellung von Poly[{α,ω-dihydroxy-<oligo(3-(R)-hydroxybuttersäure-co-3-(R)-hydroxyvaleriansäure)-ethylen-oligo(3-(R)-hydroxybuttersäure-co-3-(R)-hydroxyvaleriansäure)>-alt-(2,2,4-trimethylhexamethylen-1,6-diisocyanat)}-co-(α,ω-dihydroxy -<oligo (adipinsäure-alt-(butandiol; diethylenglykol; ethylenglykol))>-alt-(2,2,4-trimethylhexamethylen-1,6-diisocyanat)}] (Formel 9 auf Formelblatt)

25.18 g (11.00 mmol) PHB/HV-diol und 25.177 g (25.18 mmol) Diorez werden in 100 ml 1,2-Dichlorethan in einem Zweihalskolben bei 80 °C gelöst. Anschliessend wird Lösungsmittel bei einer Ölbadtemperatur von 110°C abdestilliert, bis im Kolben eine leicht viskose, noch rührbare Lösung zurückbleibt und der Wassergehalt der letzten 10 ml des Destillates bei 45 ppm liegt. Anschliessend wird die Apparatur mit Stickstoff überströmt. Nachdem die Temparatur des Ölbades auf 80 °C reduziert wurde, wird 7.5964 g (36.17 mmol) 2,2,4-Trimethylhexamethylendiisocyanat zugegeben. Um den Rührer in Bewegung zu halten, kann mit 1,2-Dichlorethan verdünnt werden. Nach 140, 167, 188 und 216 Stunden Reaktionszeit werden je 9.9, 14.3, 11.6 und 20 mg (M = 631.56 g/mol) Dibutylzinn-Dilaurat aus einer 0.016 M Stammlösung in 1,2-Dichlorethan beigegeben. Die Reaktion wird nach 220 Stunden durch Ausfällen der Polymerlösung in tiefsiedenden Petrolether abgebrochen.

### Reinigung:

Das Polymer wird erneut in 1,2-Dichlorethan gelöst und als Lösung in tiefsiedenden Petrolether ausgefällt. Danach wird das Polymer in Dioxan nochmals gelöst und durch eine G4 Nutsche filtriert, bevor das Polymer aus der Dioxanlösung in leicht basischem deionisiertem Wasser ausgefällt wird. Der pH-Wert des Wassers wird mit Natriumhydrogencarbonat auf 8-9 eingestellt. Danach wird das Polymer ein zweites Mal aus einer Dioxanlösung in deionisiertem Wasser ausgefällt.

### Beispiel 10

### Herstellung von Poly[{α,ω-dihydroxy-<oligo(3-(R)-hydroxybuttersäure-co-3-(R)-hydroxyvaleriansäure)-ethylen-oligo(3-(R)-hydroxybuttersäure-co-3-(R)-hydroxyvaleriansäure)>-alt-(2,2,4-trimethylhexamethylen-1,6-diisocyanat)}-co-{α,ω-dihydroxy-<oligo(ε-caprolacton)-ethylenoxyethylen-oligo-(ε-caprolacton)>-alt-(2,2,4-trimethylhexamethylen-1,6-diisocyanat)}] (Formel 10 auf Formelblatt)

24.58 g (10.73 mmol) PHB/HV-diol und 24.58 g (20.42 mmol) PCL-Diol werden in 100 ml 1,2-Dichlorethan in einem Zweihalskolben bei 80°C gelöst. Anschliessend wird Lösungsmittel bei einer Ölbadtemperatur von 110°C abdestilliert, bis im Kolben eine leicht viskose, noch rührbare Lösung zurückbleibt und der Wassergehalt der letzten 10 ml des Destillates bei 40 ppm liegt. Anschliessend wird die Apparatur mit Stickstoff überströmt. Nachdem die Temperatur des Ölbades auf 80°C reduziert wurde, wird 6.5427 g (31.16 mmol) 2,2,4-Trimethylhexamethylendiisocyanat zugegeben. Um den Rührer in Bewegung zu halten, kann mit 1,2-Dichlorethan verdünnt werden. Nach 162.5, 186.5 und 216 Stunden Reaktionszeit werden je 46, 16 und 16 mg (M = 631.56 g/mol) Dibutylzinn-Dilaurat aus einer 0.073 M Stammlösung in 1,2-Dichlorethan beigegeben. Die Reaktion wird nach 283 Stunden durch Ausfällen der Polymerlösung in tiefsiedendem Petrolether abgebrochen.

### Reinigung:

Das Polymer wird erneut in 1,2-Dichlorethan gelöst und als Lösung in tiefsiedendem Petrolether ausgefällt. Danach wird das Polymer in Dioxan nochmals gelöst und durch eine G4 Nutsche filtriert, bevor das Polymer aus der Dioxanlösung in leicht basischem deionisiertem Wasser ausgefällt wird. Der pH-Wert des Wassers wird mit Natriumhydrogencarbonat auf 8-9 eingestellt. Danach wird das Polymer ein zweites Mal aus einer Dioxanlösung in deionisiertem Wasser ausgefällt.

### Beispiel 11

### Herstellung von Poly[{α,ω-dihydroxy-<oligo(3-(R)-hydroxybuttersäure-co-3-(R)-hydroxyvaleriansäure)-ethylen-oligo(3-(R)-hydroxybuttersäure-co-3-(R)-hydroxyvaleriansäure)>-alt-(2,6-diisocyanatcapronsäuremethylester)}-co-{α,ω-dihydroxy-<oligo(ε-caprolacton)-ethylenoxyethylen-oligo-(ε-caprolacton)>-alt-(2,6-diisocyanatcapronsäuremethylester)}] (Formel 11 auf Formelblatt)

18.00 g (7.86 mmol) PHB/HV-diol und 18.00 g (14.95 mmol) PCL-Diol werden in 100 ml 1,2-Dichlorethan in einem Zweihalskolben bei 80°C gelöst. Anschliessend werden 20 ml des Lösungsmittel bei einer Ölbadtemperatur von 110°C abdestilliert. Danach wird das Lösungsmittel über einen mit ca. 30 g Molekularsieb A4 (4Å Porengrösse) gefüllten Soxhlet destilliert bis der Wassergehalt im Soxhlet bei 3.8 ppm liegt. Die Lösung wird durch Abdestillieren von Lösungsmittel aufkonzentriert bis im Kolben eine leicht viskose, noch rührbare Lösung zurückbleibt. Anschliessend wird die Apparatur unter einen leichten Stickstoffüberdruck gesetzt. Nachdem die Temparatur des Ölbades auf 80°C reduziert wurde, werden 4.845 g (22.83 mmol) Lysinmethylesterdiisocyanat zugegeben werden. Um den Rührer in Bewegung zu halten, kann je nach Bedarf etwas trockenes 1,2-Dichlorethan zugegeben. Nach 188.75 Stunden Reaktionszeit werden 0.138 g (0.65 mmol) Lysinmethylesterdiisocyanat zugegeben. Die Reaktion wird nach 194.25 Stunden durch Ausfällen der Polymerlösung in tiefsiedendem Petrolether abgebrochen.

### Reinigung:

Das Polymer wird in Dioxan gelöst und die Lösung durch eine G4 Nutsche filtriert, bevor das Polymer aus der Dioxanlösung in deionisiertem Wasser ausgefällt wird. Danach wird das Polymer ein weiteres Mal aus einer Dioxanlösung in deionisiertem Wasser ausgefällt.

### Beispiel 12

### Herstellung von Poly[{α,ω-dihydroxy-<oligo(3-(R)-hydroxbuttersäure-co-3-(R)-hydroxyvaleriansäure)-ethylen-oligo(3-(R)-hydroxybuttersäure-co-3-(R)-hydroxyvaleriansäure)>-alt-(2,6-diisocyanatcapronsäuremethylester)}-co-{α,ω-dihydroxy-<oligo(adipinsäure-alt-(butandiol; diethylenglykol; ethylenglykol))>-alt-(2,6-diisocyanatcapronsäuremethylester)}]

(Formel 12 auf Formelblatt)

10.02 g (4.38 mmol) PHB/HV-diol und 10.02 g (10.02 mmol) Diorez werden in 100 ml 1,2-Dichlorethan in einem Zweihalskolben bei 80°C gelöst. Anschliessend werden 20 ml des Lösungsmittels bei einer Ölbadtemperatur von 110°C abdestilliert. Danach wird das Lösungsmittel über einen mit ca. 30 g Molekularsieb A4 (4Å Porengrösse) gefüllten Soxhlet destilliert bis der Wassergehalt im Soxhlet bei 4.5 ppm liegt. Die Lösung wird durch Abdestillieren von Lösungsmittel aufkonzentriert bis im Kolben eine leicht viskose, noch rührbare Lösung zurückbleibt. Anschliessend wird die Apparatur unter einen leichten Stickstoffüberdruck gesetzt. Nachdem die Temperatur des Ölbades auf 80°C reduziert wurde, werden 3.0818 g (14.52 mmol) Lysinmethylesterdiisocyanat zugegeben. Um den Rührer in Bewegung zu halten, kann je nach Bedarf etwas trockenes 1,2-Dichlorethan zugegeben werden. Nach 76.2 Stunden Reaktionszeit werden 0.08 g (0.38 mmol) Lysinmethylesterdiisocyanat zugegeben. Die Reaktion wird nach 166.3 Stunden durch Ausfällen der Polymerlösung in tiefsiedendem Petrolether abgebrochen.

### Reinigung:

Das Polymer wird in Dioxan gelöst und die Lösung durch eine G4 Nutsche filtriert, bevor das Polymer aus der Dioxanlösung in deionisiertem Wasser ausgefällt wird. Danach wird das Polymer ein weiteres Mal aus einer Dioxanlösung in deionisiertem Wasser ausgefällt.

### Beispiel 13

### Herstellung von Poly((α,ω-dihydroxy-(oligo(3-(R)-hydroxybuttersäure-co-3-(R)-hydroxyvaleriansäure)-ethylen-oligo(3-(R)-hydroxybuttersäure-co-3-(R)-hydroxyvaleriansäure))-alt-sebacinsäure)-co-(α,ω-dihydroxy-(oligo(adipinsäure-alt-(butandiol; diethylenglykol; ethylenglykol)))-alt-sebacinsäure)) (Formel 13 auf Formelblatt)

Zu 150 ml Dichlorethan wird 10.00 g (1.00 10⁻² mol) Diorez-Diol und 10.00 g (4.35 10⁻³ mol) PHB-Diol gegeben. Das Reaktionsgemisch wird mittels azeotroper Destillation über einem Soxhlet, gefüllt mit Molekolarsieb (5 Å), unter Stickstoffatmosphäre, während 5 Stunden, bis zu einem Wassergehalt von 5 ppm, getrocknet. Die Reaktionslösung wird anschliessend auf Eisbadtemperatur abgekühlt. Im Anschluss daran wird 2.38 g (3.01 10⁻² mol, 105 %, < 15 ppm H₂O) Pyridin sowie Katalysator (Dimethylaminopyridin, 0.1 Gew.-%) zugegeben. Danach werden 3.43 g (1.44 10⁻² mol) Sebacinsäuredichlorid zur klaren Lösung gegeben. Die Reaktion wird bei 4°C bis zum Maximum des Molekulargewichtes durchgeführt. Das Reaktionsgemisch wird mit Dichlorethan gelöst und in ein mit 10 1 dest. Wasser gefülltes Gefäss gegeben. In regelmässigen Abständen wird die wässerige Lösung gewechselt. Das Polymer wird dann vom Wasser und vom Lösungsmittel befreit und bei 50°C, 200·10² Pa während 12 Stunden getrocknet. Anschliessend noch einmal in Dioxan gelöst, in Wasser ausgefällt, abfiltriert und bei 50°C im Hochvakuum getrocknet.

### Beispiel 14

### Herstellung von Poly((α,ω-dihydroxy-(oligo(3-(R)-hydroxybuttersäure-co-3-(R)-hydroxyvaleriansäure)-ethylen-oligo(3-(R)-hydroxybuttersäure-co-3-(R)-hydroxyvaleriansäure))-alt-sebacinsäure)-co-(α,ω-dihydroxy-(oligo(ε-caprolacton)-ethylenoxyethylen-oligo(ε-caprolacton))-alt-sebacinsäure)) (Formel 14 auf Formelblatt)

Zu 150 ml Dichlorethan wird 10.00 g (8.33 10⁻³ mol) PCL-Diol und 10.00 g (4.35 10⁻³ mol) PHB-Diol gegeben. Das Reaktionsgemisch wird mittels azeotroper Destillation über einem Soxhlet, gefüllt mit Molekolarsieb (5 Å), unter Stickstoffatmosphäre, während 5 Stunden, bis zu einem Wassergehalt von 5 ppm, getrocknet. Die Reaktionslösung wird anschliessend auf Eisbadtemperatur abgekühlt. Im Anschluss daran wird 2.11 g (2.66 10⁻² mol, 105 %, < 15 ppm H₂O) Pyridin sowie Katalysator (Dimethylaminopyridin, 0.1 Gew.-%) zugegeben. Danach werden 3.03 g (1.27 10⁻² mol) Sebacinsäuredichlorid zur klaren Lösung gegeben. Die Reaktion wird bei 4°C bis zum Maximum des Molekulargewichtes durchgeführt.

Das Reaktionsgemisch wird mit Dichlorethan gelöst und in ein mit 10 1 dest. Wasser gefülltes Gefäss gegeben. In regelmässigen Abständen wird die wässerige Lösung gewechselt. Das Polymer wird dann vom Wasser und vom Lösungsmittel befreit und bei 50°C, 200·10² Pa während 12 Stunden getrocknet. Anschliessend noch einmal in Dioxan gelöst, in Wasser ausgefällt, abfiltriert und bei 50°C im Hochvakuum getrocknet.

### Beispiel 15

### Herstellung von Poly ((α,ω-dihydroxy-(oligo(3-(R)-hydroxybuttersäure-co-3-(R)-hydroxyvaleriansäure)-ethylen-oligo(3-(R)-hydroxybuttersäure-co-3-(R)-hydroxyvaleriansäure))-alt-sebacinsäure)-co-(α,ω-dihydroxy-(oligo(tetramethylenoxy))-alt-sebacinsäure)) (Formel 15 auf Formelblatt)

Zu 150 ml Dichlorethan wird 10.00 g (1.54 10⁻² mol) PTHF-Diol und 10.00 g (4.35 10⁻³ mol) PHB-Diol gegeben. Das Reaktionsgemisch wird mittels azeotroper Destillation über einem Soxhlet, gefüllt mit Molekolarsieb (5 Å), unter Stickstoffatmosphäre, während 5 Stunden, bis zu einem Wassergehalt von 5 ppm, getrocknet. Die Reaktionslösung wird anschliessend auf Eisbadtemperatur abgekühlt. Im Anschluss daran wird 3.28 g (4.15 10⁻² mol, 105 %, < 15 ppm H₂O) Pyridin sowie Katalysator (Dimethylaminopyridin, 0.1 Gew.-%) zugegeben. Danach werden 4.72 g (1.97 10⁻² mol) Sebacinsäuredichlorid zur klaren Lösung gegeben. Die Reaktion wird bei 4°C bis zum Maximum des Molekulargewichtes durchgeführt.

Das Reaktionsgemisch wird mit Dichlorethan gelöst und in ein mit 10 1 dest. Wasser gefülltes Gefäss gegeben. In regelmässigen Abständen wird die wässerige Lösung gewechselt. Das Polymer wird dann vom Wasser und vom Lösungsmittel befreit und bei 50°C, 200 mbar während 12 Stunden getrocknet. Anschliessend wird es noch einmal in Dioxan gelöst, in Wasser ausgefällt, abfiltriert und bei 50°C im Hochvakuum getrocknet.

### Halbmikroansatz (Beispiele 16 - 20)

### Beispiel 16

### Herstellung von Poly [poly[α,ω-Dihydroxy-poly(3-(R)-hydroxybutyrat)-ethylen-poly(3-(R)-hydroxybutyrat)]-alt-2,2,4-trimethylhexamethylen-1,6-diisocyanat]-co-poly[α,ω-Dihydroxy-[oligo(glycolid-ran-ε-caprolacton)-ethylenoligo(glycolid-ran-ε-caprolacton)]-alt-2, 2,4-trimethylhexamethylen-1,6-diisocyanat]]

Das Polymer enthält die Segmente PHB und SG3000.

2.500g (1.1 mmol) PHB-Diol (α,ω-Dihydroxy-poly(3-(R)-hydroxybutyrat) ethylen poly(3-(R)-hydroxybutyrat)])und 2.500g (1.0 mmol) SG3000 (α,ω-Dihydroxy-[oligo(glycolidran-ε-caprolacton) ethylen oligo(glycolid-*ran*-ε-caprolacton)] werden in 70ml trockenem (Wassergehalt < 5ppm nach der Karl Fischer Methode bestimmt) 1,2-Dichlorethan bei 70°C gelöst. Dabei wird in einer Standardrückflussapparatur unter N₂ gearbeitet. Zur Trocknung des Reaktionsgemisches wird das wasserhaltige Azeotrop des Lösungsmittels in einem Soxhletextraktor über Molekularsieb 4A destilliert. Die Badtemperatur beträgt dabei 110°C. Das Molekularsieb wird sooft ausgetauscht bis ein Wassergehalt von <5ppm des abdestillierten Lösungsmittels erreicht ist. Es wird nun soviel Lösungsmittel abdestilliert bis im Kolben eine leicht viskose, noch rührbare Lösung zurückbleibt. Nach der Absenkung der Badtemperatur auf 80°C werden 0,441g (2.1 mmol) 2,2,4-Trimethylhexamethylendiisocyanat zugegeben. Um den Rührer in Bewegung zu halten, kann je nach Bedarf etwas trockenes 1,2-Dichlorethan zugesetzt werden. Die Reaktion wird nach 284 Stunden durch Ausfällen in Hexan-Fraktion beendet.

### Reinigung:

Das Polymer wird erneut in trockenem 1,2-Dichlorethan gelöst und als Lösung in Hexan-Fraktion ausgefällt.

Die Resultate von Abbauversuchen der Blockcopolymeren der Beispiele 9, 10, 11, 13, 14 und 16 sind in Tabelle 1 zusammengestellt.

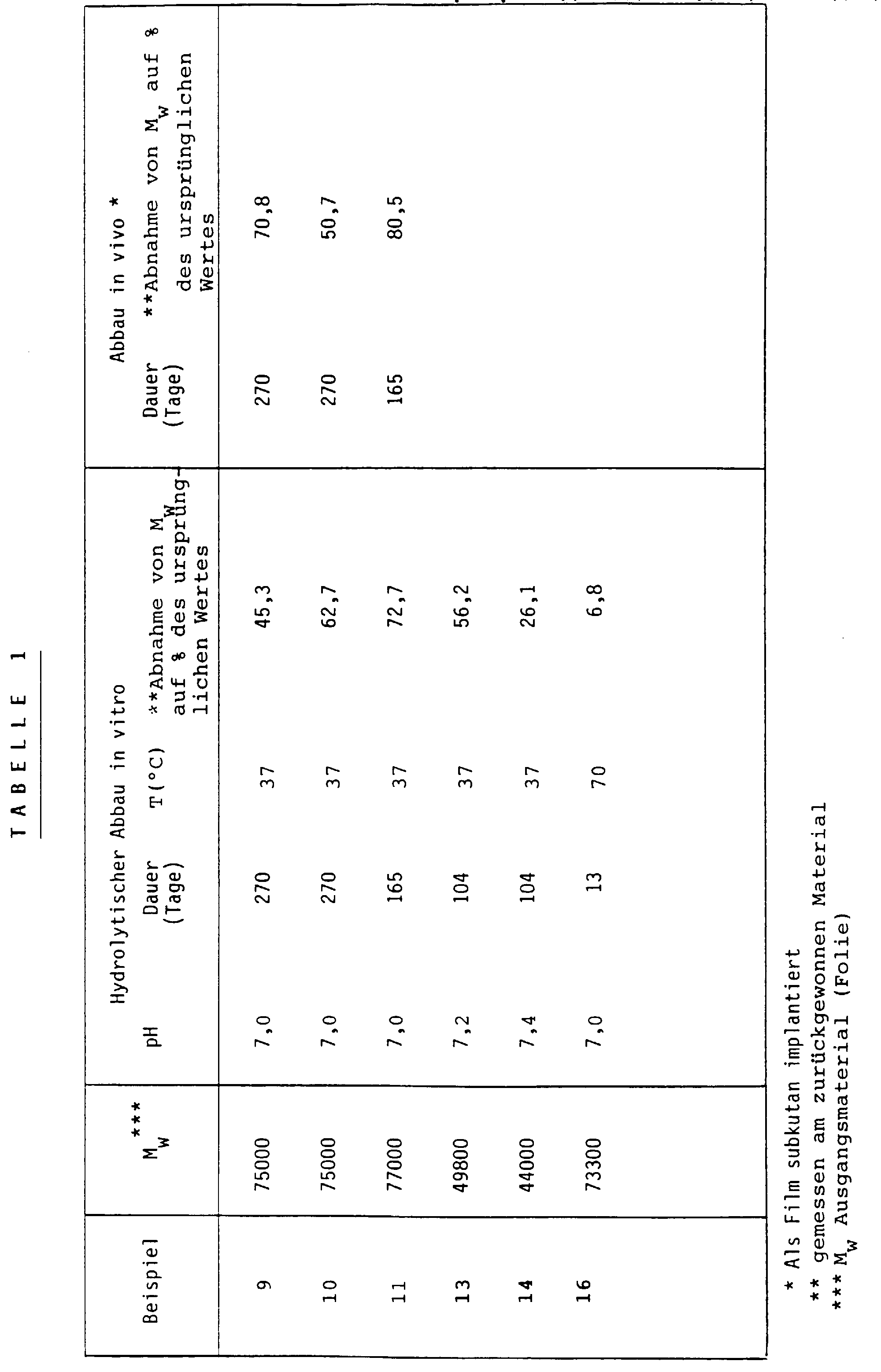

### Beispiel 17

### Herstellung von Poly [poly[α,ω-Dihydroxy-poly(3-(R)-hydroxybutyrat)-ethylen-poly(3-(R)-hydroxybutyrat)]-alt-2,2,4-trimethylhexamethylen-1,6-diisocyanat]-co-poly[α,ω-Dihydroxy-[oligo(glycolid-block-ε-caprolacton)-ethylenoligo(glycolid-block-ε-caprolacton)]-alt-2,2,4-trimethylhexamethylen-1,6-diisocyanat]]

Das Polymer enthält die Segmente PHB und HG3000.

2.500g (1.1 mmol) PHB-Diol (α,ω-Dihydroxy-poly(3-(R)-hydroxybutyrat) ethylen poly(3-(R)-hydroxybutyrat)]) und 2.500g (1.0 mmol) HG3000 (α,ω-Dihydroxy-[oligo(glycolid*block*-ε-caprolacton) ethylen oligo(glycolid-*block*-ε-caprolacton)] werden in 15ml trockenem Dimethylacetamid (Wassergehalt < 5 ppm nach der Karl Fischer Methode bestimmt) und 70ml trockenem (Wassergehalt < 5ppm nach der Karl Fischer Methode bestimmt) 1,2-Dichlorethan bei 70°C gelöst. Dabei wird in einer Standardrückflussapparatur unter N₂ gearbeitet. Zur Trocknung des Reaktionsgemisches wird das wasserhaltige Azeotrop des Lösungsmittels in einem Soxhletextraktor über Molekularsieb 4A destilliert. Die Badtemperatur beträgt dabei 110°C. Das Molekularsieb wird sooft ausgetauscht bis ein Wassergehalt von <5ppm des abdestillierten Lösungsmittels erreicht ist. Es wird nun soviel 1,2-Dichlorethan abdestilliert bis im Kolben eine leicht viskose, noch rührbare Lösung zurückbleibt. Nach der Absenkung der Badtemperatur auf 80°C werden 0.441g (2.1 mmol) 2,2,4-Trimethylhexamethylendiisocyanat zugegeben. Um den Rührer in Bewegung zu halten, kann je nach Bedarf etwas trockenes Dimethylacetamid zugesetzt werden. Die Reaktion wird nach 330 Stunden durch Ausfällen in Hexan-Fraktion beendet.

### Reinigung:

Das Polymer wird erneut in trockenem Dimethylacetamid gelöst und als Lösung in Hexan-Fraktion ausgefällt.

### Beispiel 18

### Herstellung von Poly [poly[α,ω-Dihydroxy-poly(3-(R)-hydroxybutyrat)-ethylen-poly(3-(R)-hydroxybutyrat)]-alt-2,2,4-trimethylhexamethylen-1,6-diisocyanat]-co-poly[α,ω-Dihydroxy-[oligo(glycolid-block-ε-caprolacton)-ethylenoligo(glycolid-block-ε-caprolacton)]-alt-2,2,4-trimethylhexamethylen-1,6-diisocyanat]]

Das Polymer enthält die Segmente PHB und SG500.

2.500g (1.1 mmol) PHB-Diol (α,ω-Dihydroxy-poly(3-(R)-hydroxybutyrat) ethylen poly(3-(R)-hydroxybutyrat)]) und 2.500g (5.3 mmol) SG500 (α,ω-Dihydroxy-[oligo(glycolid*block*-ε-caprolacton) ethylen oligo(glycolid-*block*-ε-caprolacton)] werden in 15ml trockenem Dimethylacetamid (Wassergehalt < 5 ppm nach der Karl Fischer Methode bestimmt) und 70ml trockenem (Wassergehalt < 5ppm nach der Karl Fischer Methode bestimmt) 1,2-Dichlorethan bei 70°C gelöst. Dabei wird in einer Standardrückflussapparatur unter N₂ gearbeitet. Zur Trocknung des Reaktionsgemisches wird das wasserhaltige Azeotrop des Lösungsmittels in einem Soxhletextraktor über Molekularsieb 4A destilliert. Die Badtemperatur beträgt dabei 110°C. Das Molekularsieb wird sooft ausgetauscht bis ein Wassergehalt von <5ppm des abdestillierten Lösungsmittels erreicht ist. Es wird nun soviel Lösungsmittel abdestilliert bis im Kolben eine leicht viskose, noch rührbare Lösung zurückbleibt. Nach der Absenkung der Badtemperatur auf 80°C werden 1.335 g (6.4 mmol) 2,2,4-Trimethylhexamethylendiisocyanat zugegeben. Um den Rührer in Bewegung zu halten, kann je nach Bedarf etwas trockenes Dimethylacetamid zugesetzt werden. Die Reaktion wird nach 330 Stunden durch Ausfällen in Hexan-Fraktion beendet.

### Reinigung:

Das Polymer wird erneut in trockenem 1,2-Dichlorethan gelöst und als Lösung in Hexan-Fraktion ausgefällt.

### Beispiel 19

### Herstellung von Poly[poly[α,ω-Dihydroxy-[oligo(glycolid-block-ε-caprolacton) -ethylen -oligo (glycolid-block-ε-caprolacton)]-alt-2,2,4-trimethylhexamethylen-1,6-diisocyanat]-co-poly[α,ω-Dihydroxy-[oligo(glycolid-ran-ε-caprolacton)-ethylen-oligo(glycolid-ran-ε-caprolacton)]-alt-2,2,4-trimethylhexamethylen-1,6-diisocyanat]]

Das Polymer enthält die Segmente HG3000 und SG3000.

Herstellung einer Stammlösung: 1.000 g (0.4 mmol) HG3000 (α,ω-Dihydroxy-[oligo(glycolid-*block*-ε-caprolacton) ethylen oligo(glycolid-*block*-ε-caprolacton)] und 1.0000g (0.4 mmol) SG3000 (α,ω-Dihydroxy-[oligo(glycolid-ran-ε-caprolacton) ethylen oligo(glycolid-*ran*-ε-caprolacton)] werden in 20.000g trockenem Diglym (Wassergehalt < 10 ppm nach der Karl Fischer Methode bestimmt) gelöst. Zur Trocknung lässt man die Lösung über Molekularsieb 4A stehen. Das Molekularsieb wird nach 24h ausgetauscht. Dieser Vorgang wird 2 bis 3 mal wiederholt.

Polymerisation: 11.000g Stammlösung werden in einen Zweihalskolben, der Bestandteil einer Standardrückflussapparatur ist, gefüllt. Nach Erreichen der Badtemperatur von 75°C werden 0.0841g (0.4 mmol) 2,2,4-Trimethylhexamethylendiisocyanat zugegeben. Um den Rührer in Bewegung zu halten, kann je nach Bedarf etwas trockenes Diglym zugesetzt werden. Die Reaktion wird nach 230 Stunden durch Ausfällen in Hexan-Fraktion beendet.

### Reinigung:

Das Polymer wird erneut in trockenem Diglym gelöst und als Lösung in Hexan-Fraktion ausgefällt.

### Beispiel 20

### Herstellung von Poly[poly[α,ω-Dihydroxy-[oligo(glycolid-block-ε-caprolacton)-ethylen-oligo(glycolid-block-ε -caprolacton)]-alt-2,2,4-trimethylhexamethylen-1,6-diisocyanat]co-poly[α,ω-Dihydroxy-[oligo(glycolid-block-ε-caprolacton)-ethylen-oligo(glycolid-block-ε-caprolacton)]-alt-2,2,4-trimethylhexamethylen-1,6-diisocyanat]]

Das Polymer enthält die Segmente HG3000-SG500.

Herstellung einer Stammlösung: 1.000g (0.4 mmol) HG3000 (α,ω-Dihydroxy-[oligo(glycolid-*block*-ε-caprolacton) ethylen oligo(glycolid-*block*-ε-caprolacton)] und 1.000g (2.1 mmol) SG500 (α,ω-Dihydroxy-[oligo(glycolid-*block*-ε-caprolacton) ethylen oligo(glycolid-*block*-ε-caprolacton)] werden in 20.000g trockenem Diglym (Wassergehalt < 10 ppm nach der Karl Fischer Methode bestimmt) gelöst. Zur Trocknung lässt man die Lösung über Molekularsieb 4A stehen. Das Molekularsieb wird nach 24h ausgetauscht. Dieser Vorgang wird 2 bis 3 mal wiederholt.

Polymerisation: 11.000g Stammlösung werden in einen Zweihalskolben, der Bestandteil einer Standardrückflussapparatur ist, gefüllt. Nach Erreichen der Badtemperatur von 75°C werden 0.2629g (1.3 mmol) 2,2,4-Trimethylhexamethylendiisocyanat zugegeben. Um den Rührer in Bewegung zu halten, kann je nach Bedarf etwas trockenes Diglym zugesetzt werden. Die Reaktion wird nach 230 Stunden durch Ausfällen in Hexan-Fraktion beendet.

### Reinigung:

Das Polymer wird erneut in trockenem Diglym gelöst und als Lösung in Hexan-Fraktion ausgefällt.

### Makroansatz (Beispiele 21 - 27)

### Beispiel 21

### Herstellung von Poly[poly[α,ω-Dihydroxy-poly(3-(R)-hydroxybutyrat)-ethylen-poly(3-(R)-hydroxybutyrat)]-alt-2,2,4-trimethylhexamethylen-1,6-diisocyanat]-co-poly[α,ω-Dihydroxy-[oligo(glycolid-ran-ε-caprolacton)-ethylen-oligo(glycolid-ran-ε-caprolacton)]-alt-2,2,4-trimethylhexamethylen-1,6-diisocyanat]]

Das Polymer enthält die Segmente PHB und SG3000.

20.000g (8.7 mmol) PHB-Diol (α,ω-Dihydroxy-poly(3-(R)-hydroxybutyrat) ethylen poly(3-(R)-hydroxybutyrat)]) und 20.000g (8.1 mmol) SG3000 (α,ω-Dihydroxy-[oligo(glycolid-*ran*-ε-caprolacton) ethylen oligo(glycolid-*ran*-ε-caprolacton)] werden in 250ml trockenem (Wassergehalt < 5ppm nach der Karl Fischer Methode bestimmt) 1,2-Dichlorethan bei 70°C gelöst. Dabei wird in einer Standardrückflussapparatur unter N₂ gearbeitet. Zur Trocknung des Reaktionsgemisches wird das wasserhaltige Azeotrop des Lösungsmittels in einem Soxhletextraktor über Molekularsieb 4A destilliert. Die Badtemperatur beträgt dabei 110°C. Das Molekularsieb wird sooft ausgetauscht bis ein Wassergehalt von <5ppm des abdestillierten Lösungsmittels erreicht ist. Es wird nun soviel Lösungsmittel abdestilliert bis im Kolben eine leicht viskose, noch rührbare Lösung zurückbleibt. Nach der Absenkung der Badtemperatur auf 80°C werden 3.5250g (16.8 mmol) 2,2,4-Trimethylhexamethylendiisocyanat zugegeben. Um den Rührer in Bewegung zu halten, kann je nach Bedarf etwas trockenes 1,2-Dichlorethan zugesetzt werden. Die Reaktion wird nach 280 Stunden durch Ausfällen in Hexan-Fraktion beendet.

### Reinigung:

Das Polymer wird erneut in trockenem 1,2-Dichlorethan gelöst und als Lösung in Hexan-Fraktion ausgefällt.

### Beispiel 22

### Herstellung von Poly[poly[α,ω-Dihydroxy-poly(3-(R)-hydroxybutyrat)-ethylen-poly(3-(R)-hydroxybutyrat)]-alt-2,2,4-trimethylhexamethylen-1,6-diisocyanat]-co-poly[α,ω-Dihydroxy-[oligo(glycolid-block-ε-caprolacton)-ethylen-oligo(glycolid-block-ε-caprolacton)]-alt-2,2,4-trimethylhexamethylen-1,6-diisocyanat]]

Das Polymer enthält die Segmente PHB und HG3000. (Formel 22 auf Formelblatt)

20.000g (8.7 mmol) PHB-Diol (α,ω-Dihydroxy-poly(3-(R)-hydroxybutyrat) ethylen poly(3-(R)-hydroxybutyrat)]) und 20.000g (8.1 mmol) HG3000 (α,ω-Dihydroxy-[oligo(glycolid-*block*-ε-caprolacton) ethylen oligo(glycolid-*block*-ε-caprolacton)) werden in 60ml trockenem Dimethylacetamid (Wassergehalt < 5 ppm nach der Karl Fischer Methode bestimmt) und 250ml trockenem (Wassergehalt < 5ppm nach der Karl Fischer Methode bestimmt) 1,2-Dichlorethan bei 70°C gelöst. Dabei wird in einer Standardrückflussapparatur unter N₂ gearbeitet. Zur Trocknung des Reaktionsgemisches wird das wasserhaltige Azeotrop des Lösungsmittels in einem Soxhletextraktor über Molekularsieb 4A destilliert. Die Badtemperatur beträgt dabei 110°C. Das Molekularsieb wird sooft ausgetauscht bis ein Wassergehalt von <5ppm des abdestillierten Lösungsmittels erreicht ist. Es wird nun soviel 1,2-Dichlorethan abdestilliert bis im Kolben eine leicht viskose, noch rührbare Lösung zurückbleibt. Nach der Absenkung der Badtemperatur auf 80°C werden 3.5327g (16.8 mmol) 2,2,4-Trimethylhexamethylendiisocyanat zugegeben. Um den Rührer in Bewegung zu halten, kann je nach Bedarf etwas trockenes Dimethylacetamid zugesetzt werden. Die Reaktion wird nach 330 Stunden durch Ausfällen in Hexan-Fraktion beendet.

### Reinigung:

Das Polymer wird erneut in trockenem 1,2-Dimethylacetamid gelöst und als Lösung in Hexan-Fraktion ausgefällt.

### Beispiel 23

### Herstellung von Poly[poly[α,ω-Dihydroxy-poly(3-(R)-hydroxybutyrat)-ethylen-poly(3-(R)-hydroxybutyrat)]-alt-2,2, 4-trimethylhexamethylen-1,6-diisocyanat]-co-poly[α,ω-Dihydroxy-[oligo(glycolid-block-ε-caprolacton)-ethylen-oligo(glycolid-block-ε-caprolacton)]-alt-2,2, 4-trimethyl-hexamethylen-1,6-diisocyanat]]

Das Polymer enthält die Segmente PHB und SG500.
(Formel 23 auf Formelblatt)

20.000g (8.7 mmol) PHB-Diol (α,ω-Dihydroxy-poly(3-(R)-hydroxybutyrat) ethylen poly(3-(R)-hydroxybutyrat)]) und 20.000g (42.1 mmol) SG500 (α,ω-Dihydroxy-[oligo(glycolid-*block*-ε-caprolacton) ethylen oligo(glycolid-*block*-ε-caprolacton)] werden in 60ml trockenem Dimethylacetamid (Wassergehalt < 5 ppm nach der Karl Fischer Methode bestimmt) und 250ml trockenem (Wassergehalt < 5ppm nach der Karl Fischer Methode bestimmt) 1,2-Dichlorethan bei 70°C gelöst. Dabei wird in einer Standardrückflussapparatur unter N₂ gearbeitet. Zur Trocknung des Reaktionsgemisches wird das wasserhaltige Azeotrop des Lösungsmittels in einem Soxhletextraktor über Molekularsieb 4A destilliert. Die Badtemperatur beträgt dabei 110°C. Das Molekularsieb wird sooft ausgetauscht bis ein Wassergehalt von <5ppm des abdestillierten Lösungsmittels erreicht ist. Es wird nun soviel 1,2-Dichlorethan abdestilliert bis im Kolben eine leicht viskose, noch rührbare Lösung zurückbleibt. Nach der Absenkung der Badtemperatur auf 80°C werden 10.68g (50.8 mmol) 2,2,4-Trimethylhexamethylendiisocyanat zugegeben. Um den Rührer in Bewegung zu halten, kann je nach Bedarf etwas trockenes Dimethylacetamid zugesetzt werden. Die Reaktion wird nach 330 Stunden durch Ausfällen in Hexan-Fraktion beendet.

### Reinigung:

Das Polymer wird erneut in trockenem Dimethylacetamid gelöst und als Lösung in Hexan-Fraktion ausgefällt.

### Beispiel 24

### Herstellung von Poly[poly[α,ω-Dihydroxy-[oligo(glycolid-block-ε-caprolacton)-ethylen-oligo(glycolid-block-ε-caprolacton)]-alt-2,2,4-trimethylhexamethylen-1,6-diisocyanat]-co-poly[α,ω-Dihydroxy-[oligo(glycolid-ran-ε-caprolacton)-ethylen-oligo(glycolid-ran-ε-caprolacton)]-alt-2,2,4-trimethylhexamethylen-1,6-diisocyanat]]

Das Polymer enthält die Segmente HG3000 und SG3000.

Herstellung einer Stammlösung: 10.000g (4.0 mmol) HG3000 (α,ω-Dihydroxy-[oligo(glycolid-*block*-ε-caprolacton) ethylen oligo(glycolid-*block*-ε-caprolacton)] und 10.000g (4.0 mmol) SG3000 (α,ω-Dihydroxy-[oligo(glycolid-*ran*-ε-caprolacton) ethylen oligo(glycolid-*ran*-ε-caprolacton)] werden in 20.000g trockenem Diglym (Wassergehalt < 10 ppm nach der Karl Fischer Methode bestimmt) gelöst. Zur Trocknung lässt man die Lösung über Molekularsieb 4A stehen. Das Molekularsieb wird nach 24h ausgetauscht. Dieser Vorgang wird 2 bis 3 mal wiederholt.

Polymerisation: 110.000g Stammlösung werden in einen Zweihalskolben, der Bestandteil einer Standardrückflussapparatur ist, gefüllt. Nach Erreichen der Badtemperatur von 75°C werden 0.8495g (4.0 mmol) 2,2,4-Trimethylhexamethylendiisocyanat zugegeben. Um den Rührer in Bewegung zu halten, kann je nach Bedarf etwas trockenes Diglym zugesetzt werden. Die Reaktion wird nach 230 Stunden durch Ausfällen in Hexan-Fraktion beendet.

### Reinigung:

Das Polymer wird erneut in trockenem Diglym gelöst und als Lösung in Hexan-Fraktion ausgefällt.

### Beispiel 25

### Herstellung von Poly[poly[α,ω-Dihydroxy-[oligo(glycolid-block-ε-caprolacton)-ethylen-oligo(glycolid-block-ε-caprolacton)]-alt-2,2,4-trimethylhexamethylen-1,6-diisocyanat]-co-poly[α,ω-Dihydroxy-[oligo(glycolid-block-ε-caprolacton)-ethylen-oligo(glycolid-block-ε-caprolacton)]-alt-2,2,4-trimethylhexamethylen-1,6-diisocyanat]]

Das Polymer enthält die Segmente HG3000-SG500.
(Formel 25 auf Formelblatt)

Herstellung einer Stammlösung: 10.000g (4.0 mmol) HG3000 (α,ω-Dihydroxy-[oligo(glycolid-*block*-ε-caprolacton) ethylen oligo(glycolid-*block*-ε-caprolacton)] und 10.000g (21.1 mmol) SG500 (α,ω-Dihydroxy-[oligo(glycolid-*block*-ε-caprolacton) ethylen oligo(glycolid-*block*-ε-caprolacton)] werden in 20.000g trockenem Diglym (Wassergehalt < 10 ppm nach der Karl Fischer Methode bestimmt) gelöst. Zur Trocknung lässt man die Lösung über Molekularsieb 4A stehen. Das Molekularsieb wird nach 24h ausgetauscht. Dieser Vorgang wird 2 bis 3 mal wiederholt.

Polymerisation: 110.000g Stammlösung werden in einen Zweihalskolben, der Bestandteil einer Standardrückflussapparatur ist, gefüllt. Nach Erreichen der Badtemperatur von 75°C werden 2.6340g (12.5 mmol) 2,2,4-Trimethylhexamethylendiisocyanat zugegeben. Um den Rührer in Bewegung zu halten, kann je nach Bedarf etwas trockenes Diglym zugesetzt werden. Die Reaktion wird nach 230 Stunden durch Ausfällen in Hexan-Fraktion beendet.

### Reinigung:

Das Polymer wird erneut in trockenem Diglym gelöst und als Lösung in Hexan-Fraktion ausgefällt.

### Beispiel 26

### Herstellung von Poly[poly[α,ω-Dihydroxy-poly(3-(R)-hydroxybutyrat)-ethylen-poly(3-(R)-hydroxybutyrat)]-alt-2,6-diisocyanatcapronsäuremethylester]-co-poly[α,ω-dihydroxy-[oligo(ε-caprolacton)-ethylen-oligo(ε-caprolacton)]-alt-2,6-diisocyanatcapronsäuremethylester]-co-poly(phenazyl-10,11-dihydroxyundecanoat-alt-2,6-diisocyanatcapronsäuremethylester)] (Formel 26 auf Formelblatt)

2.0g (0.833 mmol) α,ω-Dihydroxy-poly(3-hydroxybutyrat) (PHB-Diol) und 2.135 g (1.667 mmol) Polycaprolactondiol werden in 100 ml 1,2-Dichlorethan gelöst und über einen Soxhlet-Extraktor gefüllt mit Molekularsieb (4A-8/2) am Rückfluss getrocknet bis zu einem Wassergehalt von 4 ppm. Nach Aufkonzentrierung auf insgesamt 10 ml wird die Lösung auf Raumtemperatur abgekühlt und der Soxhlet-Extraktor durch einen Rückflusskühler mit Schutzgasaufsatz ersetzt. 1.06 g (5.0 mmol) L-Lysin-diisocyanat-methylester (LIC) wird zugegeben und die Lösung ca. 24 h bei 75 °C gerührt. Der Verlauf der Polymerisation wird mit Gelpermeationschromatographie verfolgt bis keine Molekulargewichtszunahme mehr stattfindet. Dann wird 0.84 g (2.5 mmol) Phenacyl-10,11-dihydroxyundecanoat zugegeben und die Lösung noch 48 h bei 75 °C gerührt. Anschliessend werden weitere 0.1 g (0.47 mmol) LIC zugesetzt und die Reaktionsmischung bei 75 °C gerührt, bis keine Molekulargewichtszunahme mehr erfolgt. Die Lösung wird auf Raumtemperatur abgekühlt und auf insgesamt 50 ml mit 1,2-Dichlorethan verdünnt. Das Polymer wird in Petrolether (tiefsiedende Fraktion) ausgefällt, abfiltriert und bei 40°C und 40·10² Pa im Trockenschrank 24 h getrocknet.

### Beispiel 27

### Herstellung von Poly[poly[α,ω-Dihydroxy-poly(3-(R)-hydroxybutyrat)-ethylen-poly(3-(R)-hydroxybutyrat)]-alt-2,6-diisocyanatcapronsäuremethylester]-co-poly[α,ω-dihydroxy-[oligo(ε-caprolacton)-ethylen-oligo(ε-caprolacton)]-alt-2,6-diisocyanatcapronsäuremethylester]-co-poly(10,11-dihydroxyundecansäure-alt-2,6-diisocyanatcapronsäuremethylester )]

0.9g des oben beschriebenen Polymers werden in 20 ml Essigsäure gelöst und mit 6.9 g Zinkstaub versetzt. Das Gemisch wird 21 h bei Raumtemperatur gerührt. Danach wird das Zink abfiltriert und das Polymer in Petrolether gefällt. Nach NMR Analyse wird die Phenacylschutzgruppe der Phenacyl-10,11- dihydroxyundecanoateinheit quantitativ entfernt, während das Molekulargewicht des Polymeren um 8% abnimmt.

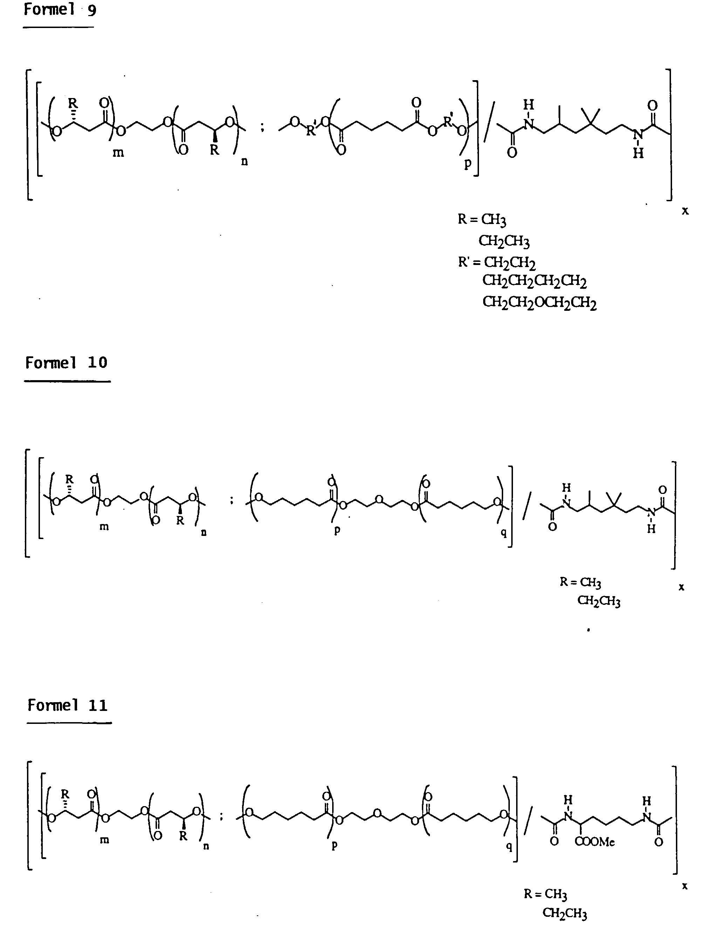

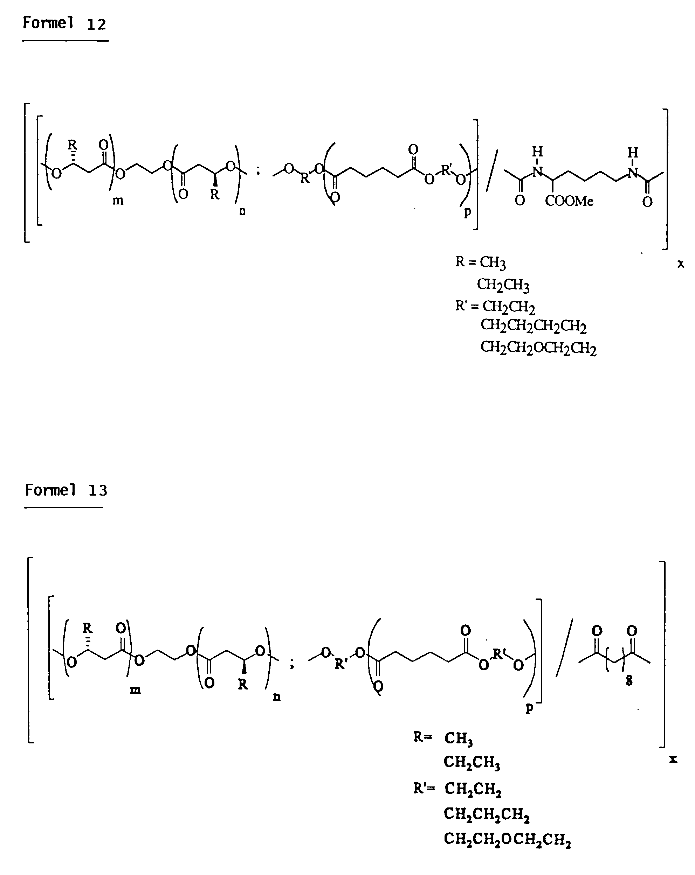

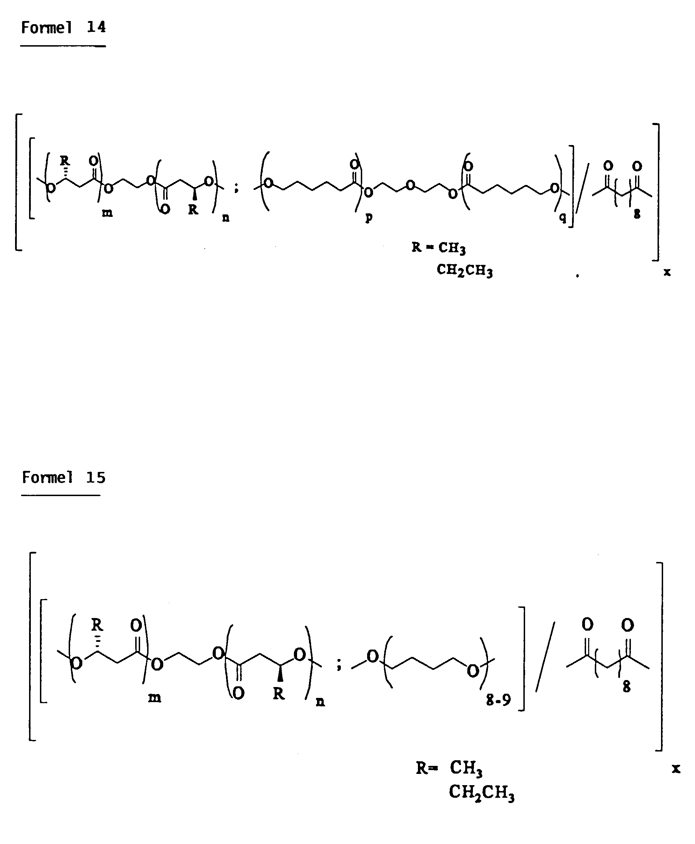

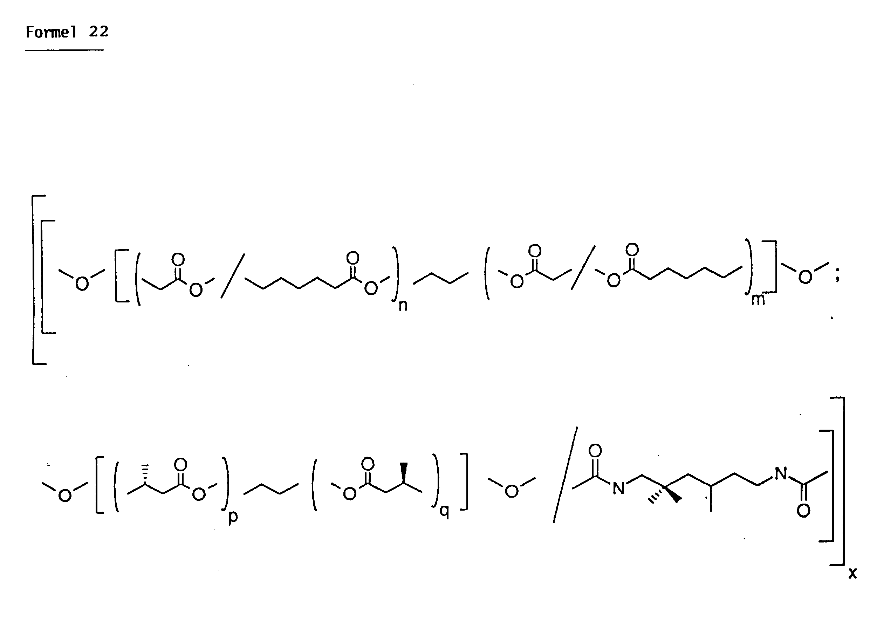

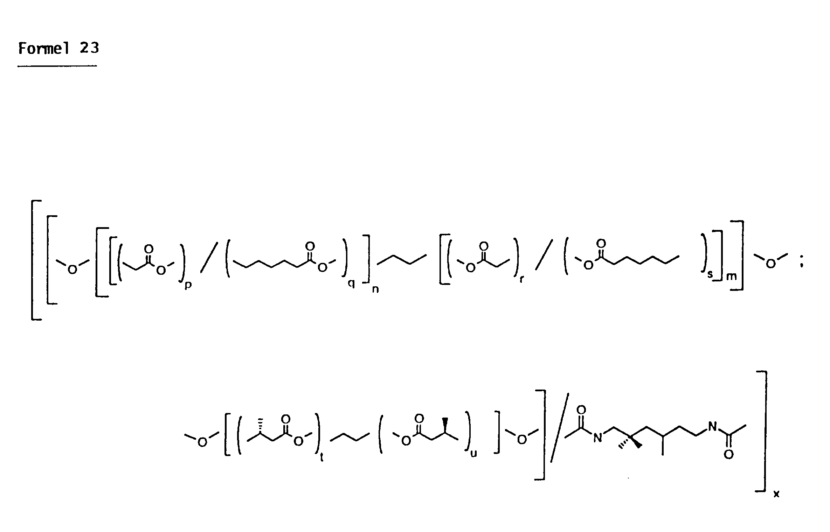

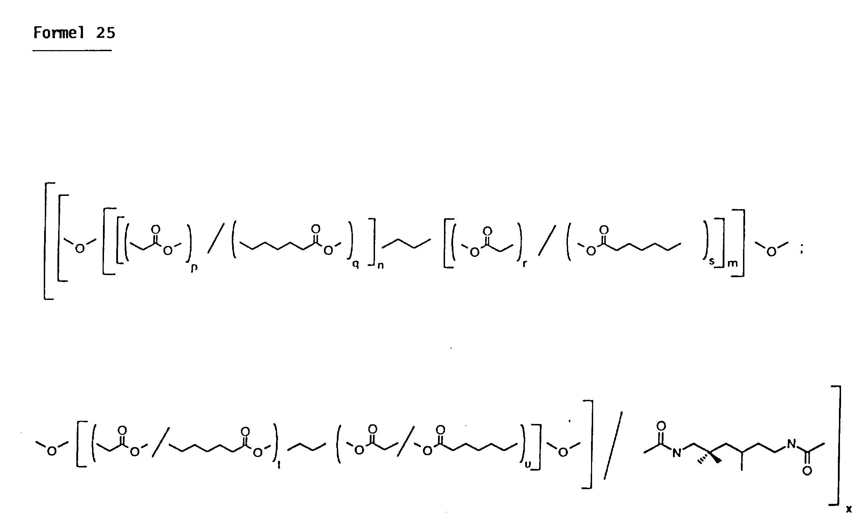

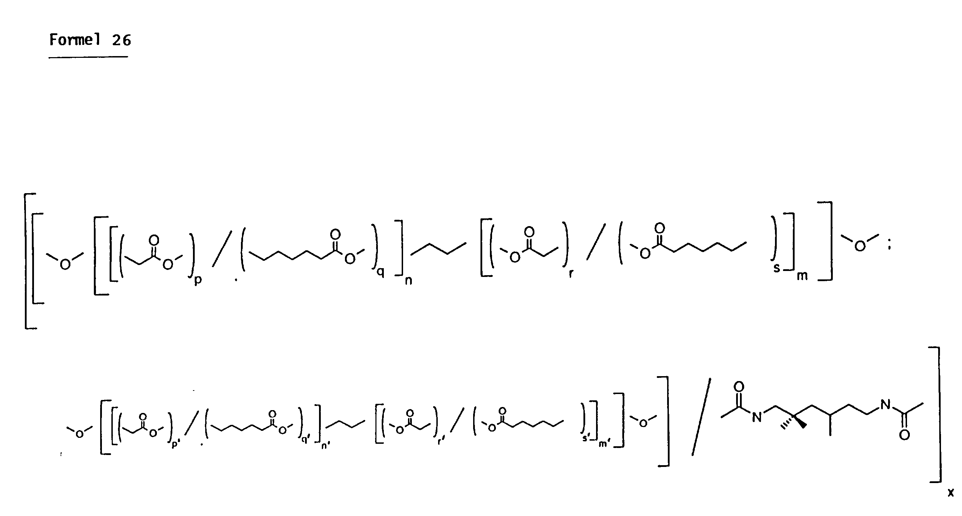

## Patentansprüche

1. Biokompatibles Multiblockcopolymer, dadurch gekennzeichnet, dass es mindestens zwei chemisch verschiedene Blockbausteine enthält und durch lineare Polykondensation eines α,ω-Dihydroxy-polyesters, der durch Transesterifikation von Poly-(R)-3-hydroxybuttersäure bzw. deren Copolymeren mit 3-Hydroxyvaleriansäure mit Ethylenglykol erhältlich ist, entweder mit einem weiteren α,ω-Dihydroxy-polyester oder mit einem α,ω-Dihydroxy-polyether das ausgewählt ist aus der Gruppe von α,ω-Dihydroxy-poly(oxytetra-methylen), α,ω-Dihydroxy-poly(oxyethylen) und Copolymeren von Ethylenglykol und Propylenglykol, mit Diisocyanat, Disäurehalogenid oder Phosgen erhältlich ist.

2. Blockcopolymer nach Anspruch 1, dadurch gekennzeichnet, dass es durch lineare Polykondensation mit Diisocyanat erhältlich ist.

3. Blockcopolymer nach Anspruch 1, dadurch gekennzeichnet, dass es durch lineare Polykondensation mit Disäurehalogenid erhältlich ist.

4. Blockcopolymer nach Anspruch 1, dadurch gekennzeichnet, dass es durch lineare Polykondensation mit Phosgen erhältlich ist.

5. Blockcopolymer nach einem der Ansprüche 1 bis 4, dadurch gekennzeichnet, dass die α,ω-Dihydroxy-polyester durch ringöffnende Polymerisation von cyclischen Estern und Lactonen, insbesondere von (L,L)-Dilactid, (D,D)-Dilactid, (D,L)-Dilactid, Diglycolid, β-(R)-Butyrolacton, β-(S)-Butyrolacton, β-rac-Butyrolacton und ε-Caprolacton und deren Gemische in Gegenwart eines aliphatischen Diols oder Polyetherdiols oder Polyesterdiols erhältlich sind.

6. Blockcopolymer nach Anspruch 1, dadurch gekennzeichnet, dass der α,ω-Dihydroxy-polyether α,ω-Dihydroxypoly(oxyethylen-co-oxypropylen) ist.

7. Blockcopolymer nach Anspruch 1, dadurch gekennzeichnet, dass der α,ω-Dihydroxy-polyester α,ω-Dihydroxypoly[adipinsäure-alt-(ethylenglykol;diethylenglykol)], α,ω-Dihydroxy-poly[adipinsäure-alt-(ethylenglykol;- propylenglykol)], α,ω-Dihydroxy-<oligo(ε-caprolacton)-ethylenoxyethylen-oligo-(ε-caprolacton)> oder Polycaprolactondiol ist.

8. Blockcopolymer nach einem der Ansprüche 1 bis 7, dadurch gekennzeichnet, dass mindestens ein α,ω-Dihydroxy-polyester eine kristallisierende Verbindung ist, die bei Raumtemperatur im Blockcopolymer kristalline Bereiche bildet.

9. Blockcopolymer nach einem der Ansprüche 1 bis 7, dadurch gekennzeichnet, dass mindestens ein α,ω-Dihydroxy-polyester eine nicht kristalline Verbindung ist, die im Blockcopolymer amorphe Bereiche bildet.

10. Blockcopolymer nach einem der Ansprüche 1 bis 7, dadurch gekennzeichnet, dass mindestens ein α,ω-Dihydroxy-polyester eine kristallisierende oder nicht kristallisierende Verbindung ist, die im Blockpolymer amorphe Bereiche bildet.

11. Blockcopolymer nach einem der vorangehenden Ansprüche, dadurch gekennzeichnet, dass es kristalline und amorphe oder mehrere kristalline oder mehrere amorphe Bereiche aufweist.

12. Blockcopolymer nach einem der vorangehenden Ansprüche, dadurch gekennzeichnet, dass es durch lineare Cokondensation weiterer niedermolekularer Verbindungen mit zusätzlichen funktionellen Gruppen erhältlich ist, wobei die funktionellen Gruppen anwendungsspezifische Funktionen erfüllen oder zur chemischen Bindung von anwendungsspezifischen Substanzen bestimmt sind.

13. Blockcopolymer nach Anspruch 12, dadurch gekennzeichnet, dass es chemisch gebundene pharmazeutische Wirkstoffe oder Diagnostika enthält.

14. Blockcopolymer nach einem der vorangehenden Ansprüche, dadurch gekennzeichnet, dass es biologisch abbaubar ist.

15. Blockcopolymer nach Anspruch 14, dadurch gekennzeichnet, dass es im menschlichen und im tierischen Körper abbaubar ist.

16. Blockcopolymer nach einem der vorangehenden Ansprüche, dadurch gekennzeichnet, dass es, bei einer Temperatur von 80°C bis 200°C vorzugsweise 100°C bis 140°C, thermoplastisch verarbeitbar ist.

17. Poly((α,ω-dihydro-(oligo(3-(R)-hydroxy-buttersäure-co-3-(R)-hydroxyvaleriansäure)-ethylen-oligo(3-(R)-hydroxybuttersäure-co-3-(R)-hydroxyvaleriansäure))-alt-sebacinsäure)-co-(α,ω-hydroxy-(oligo(tetramethylenoxy))-alt-sebacinsäure)) als Blackcopolymer nach Anspruch 1.

18. Medizinisches Implantat, gebildet im wesentlichen mit einem Blockcopolymer nach einem der vorangehenden Ansprüche.

19. Implantat nach Anspruch 18 in Form eines Rohres mit einem oder mehreren Kanälen.

20. Implantat nach Anspruch 18 oder 19, dadurch gekennzeichnet, dass es eine poröse Struktur aufweist, insbesondere zur Herstellung einfacher und zusammengesetzter biologischer Gewebe.

21. Implantat nach einem der Ansprüche 18 bis 20, dadurch gekennzeichnet, dass es zugemischte partikelförmige pharmazeutische Wirkstoffe oder Diagnostika enthält.

22. Chirurgisches Hilfsmittel, bestimmt zur Anwendung im und am Körper, gebildet mindestens teilweise mit einem Blockcopolymer nach einem der Ansprüche 1 bis 17.

23. Verwendung des Blockcopolymers nach einem der Ansprüche 1 bis 16 zur Herstellung eines medizinischen Implantats oder chirurgischen Hilfsmittels nach einem der Ansprüche 18 bis 22.

24. α,ω-Dihydroxy-polyester mit kontrollierter Mikrostruktur, random oder block, erhältlich durch ringöffnende Polymerisation von Gemischen mindestens zweier Verbindungen, ausgewählt aus der Gruppe von (L,L)-Dilactid, (D,D)-Dilactid, (D,L)-Dilactid, Diglycolid, β-(R)-Butyrolacton, β-(S)-Butyrolacton, β-rac-Butyrolacton und ε-Caprolacton, in Gegenwart eines aliphatischen Diols.

25. α,ω-Dihydroxy-polyester, erhältlich durch Transesterifikation von Poly-(R)-3-hydroxybuttersäure, bzw. deren Copolymeren mit 3-Hydroxyvaleriansäure und Ethylenglykol.

## Claims

1. Biocompatible multi-block copolymer, characterized in that it comprises at least two chemically different block units and is obtainable by linear polycondensation of an α,ω-dihydroxy-polyester, which is obtainable by transesterification of poly-(R)-3-hydroxybutyric acid or copolymers thereof with 3-hydroxyvaleric acid with ethylene glycol, either with a further α,ω-dihydroxy-polyester or with an α,ω-dihydroxy-polyether which is selected from the group consisting of α,ω-dihydroxypoly(oxytetramethylene), α,ω-dihydroxypoly(oxyethylene) and copolymers of ethylene glycol and propylene glycol, with diisocyanate, diacid halide or phosgene.

2. Block copolymer according to Claim 1, characterized in that it is obtainable by linear polycondensation with diisocyanate.

3. Block copolymer according to Claim 1, characterized in that it is obtainable by linear polycondensation with diacid halide.

4. Block copolymer according to Claim 1, characterized in that it is obtainable by linear polycondensation with phosgene.

5. Block copolymer according to one of Claims 1 to 4, characterized in that the α,ω-dihydroxy-polyesters are obtainable by ring-opening polymerization of cyclic esters and lactones, in particular (L,L)-dilactide, (D,D)-dilactide, (D,L)-dilactide, diglycolide, β-(R)-butyrolactone, β-(S)-butyrolactone, β-rac-butyrolactone and ε-caprolactone and mixtures thereof in the presence of an aliphatic diol or polyetherdiol or polyesterdiol.

6. Block copolymer according to Claim 1, characterized in that the α,ω-dihydroxy-polyether is α,ω-dihydroxypoly(oxyethylene-co-oxypropylene).

7. Block copolymer according to Claim 1, characterized in that the α,ω-dihydroxy-polyester is α,ω-dihydroxypoly[adipic acid-alt-(ethylene glycol; diethylene glycol)], α,ω-dihydroxypoly[adipic acid-alt-(ethylene glycol; propylene glycol)], α,ω-dihydroxy-<oligo(ε-caprolactone)-ethylenoxyethylene-oligo-(ε-caprolactone)> or polycaprolactonediol.

8. Block copolymer according to one of Claims 1 to 7, characterized in that at least one α,ω-dihydroxy-polyester is a crystallizing compound which forms crystalline regions in the block copolymer at room temperature.

9. Block copolymer according to one of Claims 1 to 7, characterized in that at least one α,ω-dihydroxy-polyester is a non-crystalline compound which forms amorphous regions in the block copolymer.

10. Block copolymer according to one of Claims 1 to 7, characterized in that at least one α,ω-dihydroxy-polyester is a crystallizing or non-crystallizing compound which forms amorphous regions in the block polymer.

11. Block copolymer according to one of the preceding claims, characterized in that it has crystalline and amorphous or several crystalline or several amorphous regions.

12. Block copolymer according to one of the preceding claims, characterized in that it is obtainable by linear co-condensation of further low molecular weight compounds with additional functional groups, the functional groups fulfilling functions specific to the use or being intended for chemical bonding of substances specific for the use.

13. Block copolymer according to Claim 12, characterized in that it comprises chemically bonded pharmaceutical active compounds or diagnostics.

14. Block copolymer according to one of the preceding claims, characterized in that it is biologically degradable.

15. Block copolymer according to Claim 14, characterized in that it is degradable in the human and in the animal body.

16. Block copolymer according to one of the preceding claims, characterized in that it can be melt processed at a temperature of 80°C to 200°C, preferably 100°C to 140°C.

17. Poly((α,ω-dihydroxy-(oligo(3-(R)-hydroxybutyric acid-co-3-(R)-hydroxyvaleric acid)-ethylene-oligo(3-(R)-hydroxybutyric acid-co-3-(R)-hydroxyvaleric acid))-alt-sebacic acid)-co-(α,ω-hydroxy-(oligo(tetramethyleneoxy))-alt-sebacic acid)) as block copolymer according to Claim 1.

18. Medical implant formed essentially with a block copolymer according to one of the preceding claims.

19. Implant according to Claim 18 in the form of a tube with one or more channels.

20. Implant according to Claim 18 or 19, characterized in that it has a porous structure, in particular for the production of simple and composite biological tissue.

21. Implant according to one of Claims 18 to 20, characterized in that it comprises admixed particulate pharmaceutical active compounds or diagnostics.

22. Surgical aid intended for use in and on the body, formed at least partly with a block copolymer according to one of Claims 1 to 17.

23. Use of the block copolymer according to one of Claims 1 to 16 for the production of a medical implant or surgical aid according to one of Claims 18 to 22.

24. α,ω-Dihydroxy-polyester of controlled microstructure, random or block, obtainable by ring-opening polymerization of mixtures of at least two compounds, selected from the group consisting of (L,L)-dilactide, (D,D)-dilactide, (D,L)-dilactide, diglycolide, β-(R)-butyrolactone, β-(S)-butyrolactone, β-rac-butyrolactone and ε-caprolactone, in the presence of an aliphatic diol.

25. α,ω-Dihydroxy-polyesters obtainable by transesterification of poly-(R)-3-hydroxybutyric acid or copolymers thereof with 3-hydroxyvaleric acid and ethylene glycol.

## Revendications

1. Copolymère multibloc biocompatible, caractérisé en ce qu'il renferme au moins deux motifs de bloc chimiquement différents et peut être obtenu par polycondensation linéaire d'un α,ω-dihydroxypolyester pouvant être obtenu par transestérification d'un acide poly-(R)-3-hydroxybutyrique ou de ses copolymères avec l'acide 3-hydroxyvalérique et l'éthylèneglycol, soit avec un autre α,ω-dihydroxypolyester soit avec un α,ω-dihydroxypolyéther choisi parmi le groupe constitué d'un α,ω-dihydroxypoly (oxytétraméthylène), d'un α,ω-dihydroxy-poly(oxyéthylène) et de copolymères de l'éthylèneglycol et du propylèneglycol, avec un diisocyanate, un halogénure de diacyle ou du phosgène.

2. Copolymère bloc selon la revendication 1, caractérisé en ce qu'il peut être obtenu par polycondensation linéaire avec un diisocyanate.

3. Copolymère bloc selon la revendication 1, caractérisé en ce qu'il peut être obtenu par polycondensation linéaire avec un halogénure de diacyle.

4. Copolymère bloc selon la revendication 1, caractérisé en ce qu'il peut être obtenu par polycondensation linéaire avec du phosgène.

5. Copolymère bloc selon l'une quelconque des revendications 1 à 4, caractérisé en ce que les α,ω-dihydroxypolyesters peuvent être obtenus par polymérisation décyclisante d'esters cycliques et de lactones, en particulier du (L,L)-dilactide, du (D,D)-dilactide, du (D,L)-dilactide, du diglycolide, de la β-(R)-butyrolactone, de la β-(S)-butyrolactone, de la β-rac-butyrolactone, et de la ε-caprolactone, et de leurs mélanges, en présence d'un diol ou polyétherdiol ou polyesterdiol aliphatique.

6. Copolymère bloc selon la revendication 1, caractérisé en ce que le α,ω-dihydroxypolyéther est un α,ω-dihydroxypoly(oxyéthylène-co-oxypropylène).

7. Copolymère bloc selon la revendication 1, caractérisé en ce que le α,ω-dihydroxypolyester est un α,ω-dihydroxypoly[acide adipique-alt-(éthylèneglycol ; diéthylèneglycol)], un α,ω-dihydroxy-poly[acide adipique-alt-(éthylèneglycol ; propylèneglycol)], un α,ω-dihydroxy<oligo(ε-caprolactone)-éthylène-oxyéthylène-oligo(ε-caprolactone)> ou un polycaprolactonediol.

8. Copolymère bloc selon l'une quelconque des revendications 1 à 7, caractérisé en ce qu'au moins un α,ω-dihydroxypolyester est un composé cristallisant formant des zones cristallines dans le copolymère bloc à température ambiante.

9. Copolymère bloc selon l'une quelconque des revendications 1 à 7, caractérisé en ce qu'au moins un α,ω-dihydroxypolyester est un composé non cristallin formant des zones amorphes dans le copolymère bloc.

10. Copolymère bloc selon l'une quelconque des revendications 1 à 7, caractérisé en ce qu'au moins un α,ω-dihydroxypolyester est un composé cristallisant ou non cristallisant formant des zones amorphes dans le copolymère bloc.

11. Copolymère bloc selon l'une quelconque des revendications précédentes, caractérisé en ce qu'il présente des zones cristallines et amorphes ou plusieurs zones cristallines ou plusieurs zones amorphes.

12. Copolymère bloc selon l'une quelconque des revendications précédentes, caractérisé en ce qu'il peut être obtenu par co-condensation linéaire d'autres composés à faible poids moléculaire ayant des groupes fonctionnels supplémentaires, les groupes fonctionnels remplissant des fonctions spécifiques à l'utilisation ou étant déterminés pour la liaison chimique de substances spécifiques à l'utilisation.

13. Copolymère bloc selon la revendication 12, caractérisé en ce qu'il comprend des agents diagnostiques ou des ingrédients actifs pharmaceutiques liés chimiquement.

14. Copolymère bloc selon l'une quelconque des revendications précédentes, caractérisé en ce qu'il est biodégradable.

15. Copolymère bloc selon la revendication 14, caractérisé en ce qu'il est dégradable dans des corps humains et animaux.

16. Copolymère bloc selon l'une quelconque des revendications précédentes, caractérisé en ce qu'il peut être mis en oeuvre thermoplastiquement à une température de 80°C à 200°C, de préférence de 100°C à 140°C.

17. Poly((α,ω-dihydroxy-(oligo(acide 3-(R)-hydroxybutyrique-co-acide 3-(R)-hydroxyvalérique)-éthylène-oligo(acide 3-(R)-hydroxybutyrique-co-acide 3-(R)-hydroxyvalérique))-alt-acide sébacique)-co-(α,ω-hydroxy-(oligo(tétraméthylène-oxy))-alt-acide sébacique)) en tant que copolymère bloc selon la revendication 1.

18. Implant médical formé essentiellement d'un copolymère bloc selon l'une quelconque des revendications précédentes.

19. Implant selon la revendication 18 sous la forme d'un tube avec un ou plusieurs canaux.

20. Implant selon la revendication 18 ou 19, caractérisé en ce qu'il présente une structure poreuse, en particulier pour la production de tissus biologiques simples et composites.

21. Implant selon l'une quelconque des revendications 18 à 20, caractérisé en ce qu'il comprend des agents diagnostiques ou des ingrédients actifs pharmaceutiques particulaires ajoutés.

22. Auxiliaire chirurgical, en particulier pour une utilisation dans et sur le corps, formé au moins partiellement d'un copolymère bloc selon l'une quelconque des revendications 1 à 17.

23. Utilisation du copolymère bloc selon l'une quelconque des revendications 1 à 16, pour la production d'un implant médical ou d'un auxiliaire chirurgical selon l'une quelconque des revendications 18 à 22.

24. α,ω-dihydroxypolyester ayant une microstructure régulée, statistique ou bloc, pouvant être obtenu par polymérisation décyclisante de mélanges d'au moins deux composés choisis parmi le groupe constitué du (L,L)-dilactide, du (D,D)-dilactide, du (D,L)-dilactide, du diglycolide, de la β-(R)-butyrolactone, de la β-(S)-butyrolactone, de la β-rac-butyrolactone et de la ε-caprolactone, en présence d'un diol aliphatique.

25. α,ω-dihydroxypolyester pouvant être obtenu par trans-estérification d'un acide poly-(R)-3-hydroxybutyrique ou de ses copolymères avec l'acide 3-hydroxyvalérique et l'éthylèneglycol.
